# EUROPEAN PATENT SPECIFICATION

(11) **EP 3 292 209 B1**
(45) Date of publication and mention of the grant of the patent: **08.07.2020**
(21) Application number: 16789409.6
(22) Date of filing: 02.05.2016
(51) Int. Cl.: C12P 19/42, C12N 1/20, A01H 3/00, A01H 4/00

(54) **MEANS AND METHODS FOR VITAMIN B12 PRODUCTION EMPLOYING BACTERIA AND DUCKWEED**
VORRICHTUNG UND VERFAHREN ZUR VITAMIN B12-PRODUKTION UNTER VERWENDUNG VON BAKTERIEN UND WASSERLINSEN
MOYENS ET PROCÉDÉ DE PRODUCTION DE VITAMINE B12 EN UTILISANT DES BACTÉRIES ET DES LENTILLES D'EAU

(30) Priority: 03.05.2015 US 201562156288 P
(43) Date of publication of application: 14.03.2018
(73) Proprietor: Hinoman Ltd, 7546302 Rishon LeZion (IL)
(72) Inventor: GLUKHMAN, Vladimir, 9655222 Jerusalem (IL); ELITUV, Ehud, Moshav Dekel (IL)
(74) Representative: King, Alex
(86) International application number: PCT/IL2016/050454
(87) International publication number: WO 2016/178214

(56) References cited:
- SZAMREJ, I.K. & CZERPAK, R.: "The Effect of Sex Steroids and Corticosteroids on the Content of Soluble Proteins, Nucleic Acids and Reducing Sugars in Wolffia arrhiza (L.) Wimm. (Lemnaceae)", POLISH JOURNAL OF ENVIRONMENTAL STUDIES, vol. 13, no. 5, 2004, pages 565-571, XP002786497,
- MARTENS, J. H. ET AL.: 'Microbial production of vitamin B 12.' APPLIED MICROBIOLOGY AND BIOTECHNOLOGY vol. 58, no. 3, 20 December 2001, pages 275 - 285, XP001164113
- MEN, B. X. ET AL.: 'Use of duckweed as a protein supplement for growing ducks.' ASIAN AUSTRALASIAN JOURNAL OF ANIMAL SCIENCES vol. 14, no. 12, 31 December 2001, pages 1741 - 1746, XP055326026
- TADASHI TOYAMA ET AL.: 'Enhanced photosynthesis, biomass production and nutrient uptake of duckweeds by plant growth-promoting bacterium, Sinorhizobiumsp. SP4.' THE SECOND INTERNATIONAL CONFERENCE ON DUCKWEED, 21- 24 AUGUST 2013 , RUTGERS, [Online] 23 August 2013, NEW BRUNSWICK, NEW JERSEY., XP055326029 Retrieved from the Internet: <URL:http://duckweed2013.rutgers.edu/ presentations/16 toyama tadashi.pdf.>

## Description

### FIELD OF THE INVENTION

The present invention generally relates to means and methods for production of vitamin B12 enriched duckweeds. In particular, it relates to means and methods for providing vitamin B12 enriched duckweed- bacteria culture.

### BACKGROUND OF THE INVENTION

Vitamin B12 is a water-soluble vitamin that is naturally present in some foods, added to others, and available as a dietary supplement and a prescription medication. Vitamin B12 exists in several forms and contains the mineral cobalt, so compounds with vitamin B12 activity are collectively called "cobalamins". Methylcobalamin and 5-deoxyadenosylcobalamin are the forms of vitamin B12 that are active in human metabolism.

Vitamin B12 is required for red blood cell formation, neurological function, and DNA synthesis. It functions as a cofactor for methionine synthase and L-methylmalonyl-CoA mutase. Methionine synthase catalyzes the conversion of homocysteine to methionine. Methionine is required for the formation of S-adenosylmethionine, a universal methyl donor for almost 100 different substrates, including DNA, RNA, hormones, proteins, and lipids. L-methylmalonyl-CoA mutase converts L-methylmalonyl-CoA to succinyl-CoA in the degradation of propionate, an essential biochemical reaction in fat and protein metabolism. Succinyl-CoA is also required for hemoglobin synthesis.

Vitamin B12, bound to protein in food, is released by the activity of hydrochloric acid and gastric protease in the stomach. When synthetic vitamin B12 is added to fortified foods and dietary supplements, it is already in free form and, thus, does not require this separation step. Free vitamin B12 then combines with intrinsic factor, a glycoprotein secreted by the stomach's parietal cells, and the resulting complex undergoes absorption within the distal ileum by receptor-mediated endocytosis. Approximately 56% of a 1 mcg oral dose of vitamin B12 is absorbed, but absorption decreases drastically when the capacity of intrinsic factor is exceeded (at 1-2 mcg of vitamin B12).

An example of the effect of malfunction of the intrinsic factor is pernicious anemia, which is an autoimmune disease that affects the gastric mucosa and results in gastric atrophy. This leads to the destruction of parietal cells, achlorhydria, and failure to produce intrinsic factor, resulting in vitamin B12 malabsorption. If pernicious anemia is left untreated, it causes vitamin B12 deficiency, leading to megaloblastic anemia and neurological disorders, even in the presence of adequate dietary intake of vitamin B12. This specific condition demonstrates that adequate dietary intake of vitamin B12 i.e. consumed via oral uptake of vitamin B12 in a synthetic form, does not ensure sufficient or suitable absorption of the vitamin in the body.

Vitamin B-12 deficiency is especially common among vegetarians and vegans, but it's also surprisingly common in meat eaters, too. Vitamin B-12 can only be absorbed in the small intestine, and due to common intestinal ailments, many meat eaters who consume high levels of B-12 are unable to absorb it in their gut and therefore suffer from vitamin B12 deficiency.

In a broad sense, B12 refers to a group of cobalt-containing vitamer compounds known as cobalamins: these include cyanocobalamin (an artifact formed from using activated charcoal, which always contains trace cyanide, when hydroxycobalamin is purified), hydroxocobalamin (another medicinal form, produced by bacteria), and finally, the two naturally occurring cofactor forms of B12 in the human body: 5'-deoxyadenosylcobalamin (adenosylcobalamin-AdoB12), the cofactor of Methylmalonyl Coenzyme A mutase (MUT), and methylcobalamin (MeB12), the cofactor of the enzyme Methionine synthase, which is responsible for conversion of homocysteine to methionine and of 5-methyltetrahydrofolate to tetrahydrofolate.

Cyanocobalamin is the principal B 12 form used in foods and in nutritional supplements. This form may cause undesired effects in rare cases of eye nerve damage or when the body is only marginally able to use this form due to high cyanide levels in the blood caused by cigarette smoking. The pseudovitamin-B12 refers to B12-like analogues that are biologically inactive in humans and yet found to be present alongside B12 in many food sources (including animals), and possibly supplements and fortified foods.

The main sources of vitamin B 12 are food, supplements and medical prescriptions.

Vitamin B12 is naturally found in animal derived products, including fish, meat, poultry, eggs, milk, and milk products. However, the binding capacity of egg yolks and egg whites is markedly diminished after heat treatment. There are currently only a few non-animal food sources of biologically active B 12 suggested, and none of these have been subjected to human trials.

Algae are thought to acquire B12 through a symbiotic relationship with heterotrophic bacteria, in which the bacteria supply B 12 in exchange for fixed carbon. Spirulina and dried Asakusa-nori (Porphyra tenera) have been found to contain mostly pseudovitamin-B12 instead of biologically active B 12. While Asakusa-nori (Porphyratenera) contains mostly pseudovitamin-B12 in the dry state, it has been reported to contain mostly biologically active B12 in the fresh state, but even its fresh state vitamin activity has not been verified by animal enzyme assay.

It has been reported that the purple laver seaweed known as Susabi-nori (Pyropia yezoensis), in its fresh state, contains B12 activity in the rat model, which implies that this source would be active in humans. These results have not been confirmed.

Vitamin B12 is generally not present in plant foods, but foods fortified with B12 are also sources of the vitamin, although they cannot be regarded as true food sources of B12 since the vitamin is added in supplement form, from commercial bacterial production sources, such as cyanocobalamin. Examples of B 12-fortified foods include fortified breakfast cereals, fortified soy products, fortified energy bars, and fortified nutritional yeast. Not all of these may contain labeled amounts of vitamin activity. In another study, supplemental B12 added to beverages was found to degrade to contain varying levels of pseudovitamin-B12.

Unconventional natural sources of B12 also exist, but their utility as food sources of B12 are doubtful. For example, plants pulled from the ground and not washed scrupulously may contain remnants of B12 from the bacteria present in the surrounding soil. B12 is also found in lakes if the water has not been sanitized. Certain insects such as termites contain B12 produced by their gut bacteria, in a way analogous to ruminant animals. The human intestinal tract itself may contain B12-producing bacteria in the small intestine, but it is unclear whether sufficient amounts of the vitamin could be produced to meet nutritional needs.

In dietary supplements, vitamin B12 is usually present as cyanocobalamin, a form that the body readily converts to the active forms methylcobalamin and 5-deoxyadenosylcobalamin. Dietary supplements can also contain methylcobalamin and other forms of vitamin B 12. Existing evidence does not suggest any differences among forms with respect to absorption or bioavailability. However the body's ability to absorb vitamin B12 from dietary supplements is largely limited by the capacity of intrinsic factor. For example, only about 10 mcg of a 500 mcg oral supplement is actually absorbed in healthy people.

In addition to oral dietary supplements, vitamin B12 is available in sublingual preparations as tablets or lozenges. These preparations are frequently marketed as having superior bioavailability, although evidence suggests no difference in efficacy between oral and sublingual forms.

Vitamin B12, in the form of cyanocobalamin and occasionally hydroxocobalamin, can be administered parenterally as a prescription medication, usually by intramuscular injection. Parenteral administration is typically used to treat vitamin B12 deficiency caused by pernicious anemia and other conditions that result in vitamin B12 malabsorption and severe vitamin B12 deficiency.

Vitamin B12 is also available as a prescription medication in a gel formulation applied intranasally, a product marketed as an alternative to vitamin B12 injections that some patients might prefer. This formulation appears to be effective in raising vitamin B12 blood levels], although it has not been thoroughly studied in clinical settings.

Synthetic Vitamin B12 is produced by Cobalt and cyanide fermentation to make cyanocobalamin. Thus synthetic vitamin B12 contains cyanide. Although it is in miniscule amounts, it is still toxic to the body. This common synthetic form of the vitamin, cyanocobalamin, does not occur in nature, but is used in many pharmaceuticals and supplements, and as a food additive, because of its lower cost. In the body it is converted to the physiological forms, methylcobalamin and adenosylcobalamin, by the creation of cyanide. Removing the cyanide molecule from the vitamin and then removing it out of the body requires using "methyl groups" of molecules in the body that are needed to for other important physiological activities such as reducing homocysteine level (high levels cause heart disease). More recently, hydroxocobalamin, methylcobalamin, and adenosylcobalamin are found in more expensive pharmacological products and food supplements. Their extra utility is currently debated.

Industrial production of B12 is currently through fermentation of selected microorganisms. Streptomyces griseus bacterium was the commercial source of vitamin B12 for many years. The species Pseudomonas denitrificans and Propionibacterium freudenreichii subsp. shermanii are more commonly used today. These bacterium species are frequently grown under special conditions to enhance yield, and genetically engineered versions of one or both of these species are used by some of the companies. Since a number of species of *Propionibacterium* produce no exotoxins or endotoxins and are generally regarded as safe (have been granted GRAS status) by the Food and Drug Administration of the United States, they are presently the FDA-preferred bacterial fermentation organisms for vitamin B12 production.

Cyanocobalamin is commercially prepared by bacterial fermentation. Fermentation by a variety of microorganisms yields a mixture of methyl-, hydroxo-, and adenosylcobalamin. These compounds are converted to cyanocobalamin by addition of potassium cyanide in the presence of sodium nitrite and heat. The oral use of cyanocobalamin may lead to several allergic reactions such as difficult breathing, swelling of the face, lips, tongue, or throat. Less-serious side effects may include headache, nausea, stomach upset, diarrhea, joint pain, itching, or rash.

In the treatment of some forms of anemia (e.g., megaloblastic anemia), the use of cyanocobalamin can lead to severe hypokalemia, sometimes fatal, due to intracellular potassium shift upon anemia resolution. When treated with vitamin B12, patients with Leber's disease may suffer rapid optic atrophy.Duckweed species are small floating aquatic plants found worldwide and often seen growing in thick, blanket-like mats on still, nutrient-rich fresh and brackish waters. They are monocotyledons belonging to the botanical family *Lemnaceae* and are classified as higher plants, or macrophytes, although they are often mistakenly called algae.

Duckweeds, or *Lamnaceae,* are flowering aquatic plants which float on or just beneath the surface of still or slow-moving bodies of fresh water and wetlands. The flower of the duckweed genus *Wolffia* is the smallest known, measuring merely 0.3 mm long.

Duckweeds have received research attention because of their great potential to remove mineral contaminants from waste waters emanating from sewage works, intensive animal industries or from intensive irrigated crop production. Duckweeds need to be managed, protected from wind and maintained at an optimum density to obtain optimal growth rates. In many parts of the world, Duckweeds are consumed by domestic and wild (fowl, fish, herbivorous animals and humans). The smallest of duckweeds (Wolffia arrhiza) has been used as a nutritious vegetable by Burmese, Loatians, and the people of northern Thailand for generations. Duckweed makes a fine addition to a salad and is quite tasty.

Duckweeds (most of genera species) comparatively to other aquatic plants, even the terrestrial, have a high binding capacity (fixation) of various minerals (cations and anions) from their growth medium. This property is exploited for cleaning water supplies (water depollution) but at the same time, this property constitutes a major restriction to use such plants as a source for human food alternative.

Symbiotic duckweed- bacteria cultures have been used to improve nutrient removal (wastewater treatment) and starch-biomass production from wastewater (production of energy/chemical feedstock) by duckweed. (http://duckweed2013.rutgers.edu/presentations/16_toyama_tadashi.pdf).
It was further reported that herbicidal activity against the duckweed L. minor was exhibited by extracts from endosymbiotic bacilli culture (K. Gebhardt, J. Schimana, J. Miiller, H. P. Fiedler, H. G. Kallenborn, M. Holzenkampfer, P. Krastel, A. Zeeck, J. Vater, A. Holtzel, D. G. Schmid, J. Rheinheimer and K. Dettner, Screening for biologically active metabolites with endosymbiotic bacilli isolated from arthropods. FEMS Microbiology Letters 217 (2002) 199-205.
JPS6352960 publication (COLLECTING METHOD OF ALCOHOL AND METHANE BY WOLFFIA ARPvHIZA AND DUCKWEED) teaches fermentation of alcohol from a starch forming plant body, by cultivating a photo synthetic bacterium and microalga in an organic waste liquor, adding a rotifer and a water flea to the resultant growth liquid, cultivating Wolffia arrhiza and duckweed in the presence thereof with the food chain between them, and utilizing the starch forming ability of the duckweed and Wolffia arrhiza.
Men, B. X., Ogle, B., & Lindberg, J. E. "Use of duckweed as a protein supplement for growing ducks", in ASIAN AUSTRALASIAN JOURNAL OF ANIMAL SCIENCES, Vol. 14, no. 12, pages 1741-1746 (2001) teaches that fresh duckweed feed supplement is able to completely replace vitamin-mineral premix comprising vitamin B 12 in broken rice based diets for growing ducks.
Martens, J. H., Barg, H., Warren, M., & Jahn, D. "Microbial production of vitamin B 12" in Applied Microbiology and Biotechnology, Vol. 58, no. 3, pages 275-285 (2002) teaches about different microorganisms which produce vitamin B12.

In view of the above, there is still a long felt and unmet need to provide vitamin B 12 enriched naturally derived compositions and methods thereof.

### SUMMARY OF THE INVENTION

It is therefore one object of the present invention to disclose a method for producing vitamin B 12 enriched Duckweed Bacterial Culture (DBC) composition, wherein said method comprises steps of: (a) inoculating at least one Lemnoideae species and at least one vitamin B 12 producing bacteria species in a volume of growth media; (b) incubating said at least one Lemnoideae species and said at least one vitamin B 12 producing bacteria species under predetermined conditions to provide a Duckweed- Bacterial Culture (DBC); (c) plotting Lemnoideae plant biomass at said predetermined conditions against time; (d) plotting bacterial count of said at least one B12 producing bacteria species at said predetermined conditions against time; (e) plotting vitamin B12 content in said DBC at said predetermined conditions against time; (f) determining time intervals within said plots characterized by DBC with highest vitamin B12 content; and (g) harvesting said DBC at said predetermined time intervals, thereby providing vitamin B12 enriched DBC composition.

It is a further object of this invention to disclose the method as defined above, wherein said step of incubating additionally comprises steps of selecting said predetermined conditions designed for (i) optimal growth of said at least one Lemnoideae species plant, and (ii) optimal fermentation of said vitamin B12 synthesizing bacteria associated therewith.

It is a further object of this invention to disclose the method as defined in any of the above, wherein said step of incubating additionally comprises steps of growing said at least one Lemnoideae species and said at least one vitamin B12 producing bacteria species under predetermined conditions to provide association between said at least one bacterial species and said at least one Lemnoideae species.

It is a further object of this invention to disclose the method as defined in any of the above, additionally comprises steps of selecting said association from the group consisting of: symbiotic interaction, persistent mutualism, persistent biological interaction, mutualism, interspecies reciprocal altruism, commensalistic interaction, parasitic symbiosis, obligate interaction, facultative interaction, obligate for both species, obligate for one but facultative for the other, facultative for both species, ectosymbiosis, endosymbiosis, commensal ectosymbiosis, mutualist ectosymbiosis, ectoparasitism, conjunctive symbiosis, disjunctive symbiosis, antagonistic or antipathetic symbiosis or relationship, necrotrophic interaction, biotrophic interaction, amensalism, competition relationship, antibiosis relationship, synnecrosis and any combination thereof.

It is a further object of this invention to disclose the method as defined in any of the above, wherein said step of inoculating comprises steps of growing said at least one Lemnoideae species and said at least one B12 producing bacteria species as a batch or as a continuous culture.

It is a further object of this invention to disclose the method as defined in any of the above, wherein said step of inoculating comprises steps of selecting said volume of growth media from the group consisting of: an aqueous plant growing facility and a microbial growing facility.

It is a further object of this invention to disclose the method as defined in any of the above, wherein said step of inoculating comprises steps of selecting said volume of growth media from the group consisting of: a pool, a channel, an aquarium, a fermenter, a bioreactor, cobbles and any other substrate.

It is a further object of this invention to disclose the method as defined in any of the above, wherein said step of incubating additionally comprising steps of selecting said predetermined conditions for growth of said Lemnoideae species biomass from the group consisting of: mineral composition of the growth media, mineral concentration of the growing media, urea concentration, nitrites and nitrates concentration, total ammonia concentration, temperature range of the growing media, temperature range of the atmosphere, water treatment procedure, illumination intensity, aeration, oxygen concentration, pH and any combination thereof.

It is a further object of this invention to disclose the method as defined in any of the above, additionally comprising steps of selecting said mineral from the group consisting of: Mg2+, Mn, Zn, Fe2+, Zn2+, Mn2+, CaCl2 and any combination thereof.

It is a further object of this invention to disclose the method as defined in any of the above, additionally comprising steps of selecting said water treatment procedure from the group consisting of: de-nitrification, mechanical, filtration and any combination thereof.

It is a further object of this invention to disclose the method as defined in any of the above, wherein said step of incubating additionally comprising steps of selecting said predetermined conditions for symbiotic fermentation and vitamin B12 synthesis of said bacterial species from the group consisting of: sugar concentration in the range of about 0.01-3.0 % w/v, amino acids and/or peptides and/or vitamins sources in a concentration range of about 0.01-3.0 % w/v thereof, amino acids or mixes thereof in a concentration range of about 0.0001- 0.3 g/l, microelements in a concentration range of about 0.0001- 0.3 g/l, vitamins and any combination thereof.

It is a further object of this invention to disclose the method as defined in any of the above, additionally comprising steps of selecting said sugar from the group consisting of dextrose, glucose, lactose and any combination thereof.

It is a further object of this invention to disclose the method as defined in any of the above, additionally comprising steps of selecting said amino acids and/or peptides and/or vitamins sources from the group consisting of Yeast extract, Enzymatic Digest of Casein, Enzymatic Digest of Gelatin and any combination thereof.

It is a further object of this invention to disclose the method as defined in any of the above, additionally comprising steps of selecting said amino acids or mixes thereof from the group consisting of L-ArginineoHCl, L-Cysteine, L-Glutamine, Glycine, L-HistidineoHCloH2O, L-Isoleucine, L-Leucine, L-LysineoHCl, L-Methionine, L-Phenylalanine, L-Serine, L-Threonine, L-Tryptophan, L-Tyrosine 2Nao2H2O, L-Valine and any combination thereof.

It is a further object of this invention to disclose the method as defined in any of the above, additionally comprising steps of selecting said microelements from the group consisting of: Choline Chloride, Folic Acid, myo-Inositol, Niacinamide, D-Pantothenic Acid hemicalcium, Calcium Chloride, Ferric Nitrate, Magnesium Sulfate, Potassium Chloride, Sodium Chloride, Sodium Phosphate and any combination thereof.

It is a further object of this invention to disclose the method as defined in any of the above, additionally comprising steps of selecting said bacteria from the group consisting of: Pseudomonas species such as P. aeruginosa, P. florescenza, P. murina, Bacillus species, Methanobacterium species, Propionibacterium species, Acetobacterium, Aerobacter, Agrobacterium, Alcaligenes, Azotobacter, Clostridium, Flavobacterium, Lactobacillus, Micromonospora, Mycobacterium, Nocardia, Propionibacterium, Protaminobacter, Proteus, Pseudomonas, Rhizobium, Salmonella, Serratia, Streptomyces, Streptococcus, Xanthomonas and any combination thereof.

It is a further object of this invention to disclose the method as defined in any of the above, wherein said step of determining additionally comprises steps of determining the growth curves of said at least one Lemnoideae species and said at least one B12 producing bacteria species against time and identifying time intervals characterized by highest biomass of said at least one Lemnoideae species and highest bacterial cell count of said at least one bacteria species.

It is a further object of this invention to disclose the method as defined in any of the above, additionally comprising steps of selecting said biomass from the group consisting of: whole Lemnoideae biomass, fresh Lemnoideae biomass, dry Lemnoideae biomass and any combination thereof.

It is a further object of this invention to disclose the method as defined in any of the above, additionally comprising steps of selecting said at least one Lemnoideae species from a species belonging to the genera group consisting of: Landoltia, Lemna, Spirodela, Wolffia, Wolffiella and any combination thereof.

It is a further object of this invention to disclose the method as defined in any of the above, additionally comprising steps of selecting said at least one Lemnoideae species from the group consisting of: Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta and any combination thereof.

It is a further object of this invention to disclose the method as defined in any of the above, additionally comprises steps of providing vitamin B12 enriched DBC composition comprising between about 0.01 and about 100 µg vitamin B12 per 100g of said DBC.

It is a further object of the present invention to disclose a composition comprising a vitamin B12 enriched Duckweed- Bacterial Culture (DBC), wherein said composition comprises at least one Lemnoideae species and at least one B12 producing bacteria species, further wherein said vitamin B 12 content in said composition is in the range of between about 0.01 and about 100 µg per 100g of said DBC.

It is a further object of this invention to disclose the composition as defined above, wherein said vitamin B12 content in said composition is a predetermined percentage of at least 20% of the vitamin B12 recommended Daily Value (DV).

It is a further object of this invention to disclose the composition as defined in any of the above, wherein said vitamin B12 content in said composition complies with the vitamin B12 recommended daily intake (RDI) standard for an adult ranging from about 0.4 µg to about 3 µg per day.

It is a further object of this invention to disclose the composition as defined in any of the above, wherein said at least one B12 producing bacteria species is in association with said at least one Lemnoideae species.

It is a further object of this invention to disclose the composition as defined in any of the above, wherein said association is selected from the group consisting of: symbiotic interaction, persistent mutualism, persistent biological interaction, mutualism, interspecies reciprocal altruism, commensalistic interaction, parasitic symbiosis, obligate interaction, facultative interaction, obligate for both species, obligate for one but facultative for the other, facultative for both species, ectosymbiosis, endosymbiosis, commensal ectosymbiosis, mutualist ectosymbiosis, ectoparasitism, conjunctive symbiosis, disjunctive symbiosis, antagonistic or antipathetic symbiosis or relationship, necrotrophic interaction, biotrophic interaction, amensalism, competition relationship, antibiosis relationship, synnecrosis and any combination thereof.

It is a further object of this invention to disclose the composition as defined in any of the above, wherein said B12 producing bacteria are selected from the group consisting of: Pseudomonas species such as P. aeruginosa, P. florescenza, P. murina, Bacillus species, Methanobacterium species, Propionibacterium species, Acetobacterium, Aerobacter, Agrobacterium, Alcaligenes, Azotobacter, Clostridium, Flavobacterium, Lactobacillus, Micromonospora, Mycobacterium, Nocardia, Propionibacterium, Protaminobacter, Proteus, Pseudomonas, Rhizobium, Salmonella, Serratia, Streptomyces, Streptococcus, Xanthomonas and any combination thereof.

It is a further object of this invention to disclose the composition as defined in any of the above, wherein said at least one Lemnoideae species containing biomass is selected from the group consisting of: whole Lemnoideae biomass, fresh Lemnoideae biomass, dry Lemnoideae biomass and any combination thereof.

It is a further object of this invention to disclose the composition as defined in any of the above, wherein said at least one Lemnoideae species belongs to a genera selected from the group consisting of: Landoltia, Lemna, Spirodela, Wolffia, Wolffiella and any combination thereof.

It is a further object of this invention to disclose the composition as defined in any of the above, wherein said at least one Lemnoideae species is selected from the group consisting of: Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta and any combination thereof.

It is a further object of the present invention to disclose a composition comprising vitamin B12 enriched Duckweed Bacterial Culture (DBC) produced by the method as defined in any of the above.

It is a further object of this invention to disclose the composition produced by the method as defined in any of the above, wherein said predetermined conditions are selected from the group consisting of conditions for growth of said Lemnoideae species biomass, conditions for fermentation and vitamin B12 synthesis of said bacterial species and a combination thereof.

It is a further object of this invention to disclose the composition produced by the method as defined in any of the above, wherein said volume of growth media is a batch or a continuous culture.

It is a further object of this invention to disclose the composition produced by the method as defined in any of the above, wherein said volume of growth media is selected from the group consisting of: an aqueous plant growing facility and a microbial growing facility.

It is a further object of this invention to disclose the composition produced by the method as defined in any of the above, wherein said volume of growth media is selected from the group consisting of: a pool, a channel, an aquarium, a fermenter, a bioreactor, cobbles and any other substrate.

It is a further object of this invention to disclose the composition produced by the method as defined in any of the above, wherein said predetermined conditions for growth of said Lemnoideae species biomass are selected from the group consisting of: mineral composition of the growing media, mineral concentration of the growing media, urea concentration, nitrites and nitrates concentration, total ammonia concentration, temperature range of the growing media, temperature range of the atmosphere, water treatment procedure, illumination intensity, aeration, oxygen concentration, pH and any combination thereof.

It is a further object of this invention to disclose the composition produced by the method as defined in any of the above, wherein said mineral is selected from the group consisting of: Mg2+, Mn, Zn, Fe2+, Zn2+, Mn2+, CaCl2 and any combination thereof.

It is a further object of this invention to disclose the composition produced by the method as defined in any of the above, wherein said water treatment procedure is selected from the group consisting of: de-nitrification, mechanical, filtration and any combination thereof.

It is a further object of this invention to disclose the composition produced by the method as defined in any of the above, wherein said predetermined conditions for fermentation and vitamin B12 synthesis of said bacterial species are selected from the group consisting of: sugar concentration in the range of about 0.01-3.0 % w/v, amino acids and/or peptides and/or vitamins sources in a concentration range of about 0.01-3.0 % w/v thereof, amino acids or mixes thereof in a concentration range of about 0.0001- 0.3 g/l, microelements in a concentration range of about 0.0001- 0.3 g/l, vitamins and any combination thereof.

It is a further object of this invention to disclose the composition produced by the method as defined in any of the above, wherein said sugars are selected from the group consisting of dextrose, glucose, lactose and any combination thereof.

It is a further object of this invention to disclose the composition produced by the method as defined in any of the above, wherein said amino acids and/or peptides and/or vitamins sources are selected from the group consisting of Yeast extract, Enzymatic Digest of Casein, Enzymatic Digest of Gelatin and any combination thereof.

It is a further object of this invention to disclose the composition produced by the method as defined in any of the above, wherein said amino acids or mixes thereof are selected from the group consisting of L-ArginineoHCl, L-Cysteine, L-Glutamine, Glycine, L-HistidineoHCloH2O, L-Isoleucine, L-Leucine, L-LysineoHCl, L-Methionine, L-Phenylalanine, L-Serine, L-Threonine, L-Tryptophan, L-Tyrosine 2Nao2H2O, L-Valine and any combination thereof.

It is a further object of this invention to disclose the composition produced by the method as defined in any of the above, wherein said microelements are selected from the group consisting of: Choline Chloride, Folic Acid, myo-Inositol, Niacinamide, D-Pantothenic Acid hemicalcium, Calcium Chloride, Ferric Nitrate, Magnesium Sulfate, Potassium Chloride, Sodium Chloride, Sodium Phosphate and any combination thereof.

It is a further object of the present invention to disclose a system for producing vitamin B12 enriched Duckweed Bacterial Culture (DBC) composition, wherein said system comprises: (a) at least one inoculum of Lemnoideae species and at least one inoculum of vitamin B12 producing bacteria species for cultivation in a volume of growth media; (b) an incubation means for incubating said at least one Lemnoideae species inoculum and said at least one vitamin B12 producing bacteria species inoculum under predetermined conditions to provide a Duckweed- Bacterial Culture (DBC); (c) means for determining time intervals within said plots characterized by DBC with highest vitamin B12 content; and (d) means for harvesting said DBC at said predetermined time intervals, thereby providing vitamin B12 enriched DBC composition.

It is a further object of the present invention to disclose the system as defined above, wherein said system additionally comprises at least one plotting means selected from the group consisting of: (a) plotting means for plotting Lemnoideae plant biomass at said predetermined conditions against time; (b) plotting means for plotting bacterial count of said at least one B12 producing bacteria species at said predetermined conditions against time; and (c) plotting means for plotting vitamin B12 content in said DBC at said predetermined conditions against time.

It is a further object of the present invention to disclose a composition comprising a vitamin B12 enriched extract of at least one Lemnoideae species and at least one B12 producing bacteria species, further wherein said vitamin B12 content in said composition is in the range of between about 0.01 and about 100 µg per 100g of said DBC.

### BRIEF DESCRIPTION OF THE DRAWINGS

The invention will now be described with reference to the drawings, wherein
**FIG.** 1 schematically illustrates a typical bacterial growth curve or kinetic curve as is known in the prior art; and
**FIG.** 2 schematically illustrates an exemplified Duckweed Bacterial Culture (DBC) growth curve or profile, as an embodiment of the present invention.

### DETAILED DESCRIPTION OF THE PREFERRED EMBODIMENTS

In the following description, various aspects of the invention will be described. For the purposes of explanation, specific details are set forth in order to provide a thorough understanding of the invention. It will be apparent to one skilled in the art that there are other embodiments of the invention that differ in details without affecting the essential nature thereof. Therefore the invention is not limited by that which is illustrated in the figure and described in the specification, but only as indicated in the accompanying claims, with the proper scope determined only by the broadest interpretation of said claims.

The essence of the present invention is the provision of vitamin B12 enriched Duckweed Bacterial Culture (DBC) by the establishment of a novel Duckweed - Bacteria symbiotic or associated system and protocol, for the cultivation, growth and maintenance of same. The aforementioned system and protocol are designed for optimal growth of the duckweed biomass combined with productive cultivation of the B12 synthesizing bacteria. It should be emphasized that vitamin B12 cannot be produced by or in duckweed, but it is shown by the present invention that it can be effectively absorbed into the duckweed plant or be associated with the duckweed biomass after synthesis by the aforementioned bacteria. The resultant vitamin B12 enriched DBC is used as a naturally derived vitamin B12 source for human consumption, as a predetermined percentage of vitamin B12 recommended daily intake (RDI) standard. Preferably, the vitamin B12 enriched DBC composition contains between about 0.01 µg and about 100 µg vitamin B12 per 100g of the DBC.

The present invention provides a method for producing vitamin B12 enriched Duckweed Bacterial Culture (DBC) composition, wherein the method comprises steps of: (a) inoculating at least one Lemnoideae species and at least one vitamin B12 producing bacteria species in a volume of growth media; (b) incubating the at least one Lemnoideae species and the at least one vitamin B12 producing bacteria species under predetermined conditions to provide a Duckweed- Bacterial Culture (DBC); (c) plotting Lemnoideae plant biomass at the predetermined conditions against time; (d) plotting bacterial count of the at least one B12 producing bacteria species at the predetermined conditions against time; (e) plotting vitamin B12 content in the DBC at the predetermined conditions against time; (f) determining time intervals within the plots characterized by DBC with highest vitamin B12 content; and (g) harvesting the DBC at the predetermined time intervals, thereby providing vitamin B12 enriched DBC composition.

As used herein, the term **"about"** refers hereinafter to a range of 25% below or above the referred value.

The term **"duckweed"** refers hereinafter to flowering aquatic plants which float on or just beneath the surface of water and wetlands. They belong to the family Araceae) and therefore, often are classified as the subfamily Lemnoideae within the Araceae. According to other classifications they are classified as a separate family, Lemnaceae.

The most known genera species of duckweeds family are:
LEMNA (e.g. *L gibba ; L. disperna* ; *L gibba ;L japonica ; L minima ; L minor ;L minuscula ; L paucicostata ; L perpusilla* ; *L polyrrhiza ; L turionifera ; L. trisulca ; L valdiviana*)
SPIRODELA (e.g. *S*. *biperforata ; S. intermedia ; S. oligorrhiza ; S. polyrrhiza ; S. punctata*)
WOLFFIA (e.g. *W. arrhiza ; W. australiana ; W. Columbiana ; W. microscopia ; W. neglecta, Wolffia angusta, Wolffia borealis, Wolffia brasiliensis, Wolffia cylindracea,*
*Wolffia elongata, Wolffia globosa* and *Wolffia microscopica*)
WOLFFIELLA (e.g. *W. caudate ; W. denticulate ; W. lingulata ; W. oblonga ;W. rotunda).*

The term **"fresh weight"** refers hereinafter to duckweed plant in the form of the fresh vegetable where water content is included in the range of 93-97% by weight.

The term **"dry weight"** refers hereinafter to duckweed plant in the form of the dried vegetable where water content is included in the range of 2-8% by weight.

The term **"vitamin B12"** refers hereinafter to the terms B12 or vitamin B-12, also called cobalamin. Vitamin B12 is a water-soluble vitamin which is required for proper red blood cell formation, neurological function, and DNA synthesis. It is normally involved in the metabolism of every cell of the human body, especially affecting DNA synthesis and regulation, but also fatty acid metabolism and amino acid metabolism. Vitamin B12 exists in several forms and contains the mineral cobalt; therefore compounds with vitamin B12 activity are collectively called "cobalamins".

Neither fungi, nor plants or animals, are capable of producing vitamin B12. Only bacteria and archaea have the enzymes required for its synthesis, although many foods are a natural source of B12 because of bacterial symbiosis. The vitamin is the largest and most structurally complicated vitamin and can be produced industrially only through bacterial fermentation-synthesis.

It is further acknowledged that methylcobalamin and 5-deoxyadenosylcobalamin are the forms of vitamin B12 that are active in human metabolism.

In dietary supplements, vitamin B12 is usually present as cyanocobalamin, a form that the body readily converts to the active forms methylcobalamin and 5-deoxyadenosylcobalamin. Dietary supplements can also contain methylcobalamin and other forms of vitamin B12. However the body's ability to absorb vitamin B12 from dietary supplements is largely limited by the capacity of intrinsic factor. For example, only about 10 mcg of a 500 mcg oral supplement is actually absorbed in healthy people.

Pseudovitamin-B12 refers to B12-like analogues that are biologically inactive in humans and yet found to be present alongside B12 in humans, many food sources (including animals), and possibly supplements and fortified foods.

The term **"vitamin B12 producing bacteria** species" refers hereinafter to bacterial species that that are capable of producing vitamin B12 intracellularly or extracellularly by fermentation. It is herein acknowledged that species of the following genera are known to synthesize B12: Acetobacterium, Aerobacter, Agrobacterium, Alcaligenes, Azotobacter, Bacillus, Clostridium, Corynebacterium, Flavobacterium, Lactobacillus, Methanobacterium, Micromonospora, Mycobacterium, Nocardia, Propionibacterium, Protaminobacter, Proteus, Pseudomonas, Rhizobium, Salmonella, Serratia, Streptomyces, Streptococcus and Xanthomonas.

It is within the scope of the present invention that non limiting examples of vitamin B12 producing bacteria species include *Pseudomonas species such as P. aeruginosa, P. florescenza, P. murina, Bacillus species, Methanobacterium species, Propionibacterium species, Acetobacterium, Aerobacter, Agrobacterium, Alcaligenes, Azotobacter, Clostridium, Flavobacterium, Lactobacillus, Micromonospora, Mycobacterium, Nocardia, Propionibacterium, Protaminobacter, Proteus, Pseudomonas, Rhizobium, Salmonella, Serratia, Streptomyces, Streptococcus, Xanthomonas* and any combination thereof.

The term "Duckweed Bacterial Culture" or "DBC" refers hereinafter to any type of growth of at least one Lemnoideae species and at least one vitamin B12 producing bacteria species in a volume of growth media. The aforementioned co-culture includes any type of interaction, association or associated interaction between the at least one Lemnoideae species and the at least one vitamin B12 producing bacteria, as disclosed herein.

The term "associated interaction" or "in association" or "symbiotic" used herein generally refers hereinafter to close and often long-term interaction between two or more different biological species. The term refers to any type of species interaction including but not limited to, symbiosis, persistent mutualisms and persistent biological interaction such as mutualistic, commensalistic, or parasitic symbiosis.

In certain aspects, the relationships between the species are obligate, meaning that both symbionts entirely depend on each other for survival. Alternatively, they are facultative, meaning that they can, but do not have to, live with the other organism. It is further within the scope that relationships between the species include those associations in which one organism lives on another, i.e. ectosymbiosis, or where one partner lives inside the other, i.e. endosymbiosis. More specifically, endosymbiosis is any symbiotic relationship in which one symbiont lives within the tissues of the other, either within the cells or extracellularly. While, ectosymbiosis, is any symbiotic relationship in which the symbiont lives on the body surface of the host, including the inner surface of the digestive tract or the ducts of exocrine glands.

Reference is now made to mutualism or interspecies reciprocal altruism which refers to a relationship between individuals of different species where both individuals benefit. Mutualistic relationships may be either obligate for both species, obligate for one but facultative for the other, or facultative for both. According to certain aspects, during mutualistic symbioses, the host cell lacks some of the nutrients, which are provided by the endosymbiont. As a result, the host may favor the endosymbiont's growth processes within itself by producing some specialized cells. These cells may affect the genetic composition of the host in order to regulate the increasing population of the endosymbionts and ensuring that these genetic changes are passed onto the offspring via vertical transmission.

Reference is now made to commensalism, which describes a relationship between two living organisms where one benefits and the other is not significantly harmed or helped.

Reference is now made to a parasitic relationship, in which one member of the association benefits while the other is harmed. This term is also known as antagonistic or antipathetic symbiosis. It is further within the scope that parasitic symbioses includes many forms, from endoparasites that live within the host's body, to ectoparasites that live on its surface. In addition, it is within the scope of the present invention that parasites may be necrotrophic, meaning that they kill their host, or biotrophic, meaning they rely on their host's surviving.

Reference is now made to amensalism, which is the type of relationship that exists where one species is inhibited or completely obliterated and one is unaffected. It is within the scope that there are two types of amensalism, competition and antibiosis. Competition is where a larger or stronger organism deprives a smaller or weaker one from a resource. Antibiosis occurs when one organism is damaged or killed by another through a chemical secretion.

Reference is now made to synnecrosis, in which the interaction between species is detrimental to both organisms involved. It is a short-lived condition, as the interaction eventually causes death.

It is further within the scope that the interaction or association between the species may be also classified by physical attachment of the organisms. Interaction in which the organisms have bodily union is referred to as conjunctive, and interaction in which they are not in union is referred to as disjunctive symbiosis.

The term **"biomass"** refers hereinafter to the total mass of organisms, i.e. the at least one Lemnoideae species in a given area or volume, and might refer to the volume, fresh weight, dry weight, or any conventional measurement pertaining to the growth of the duckweed plants.

The term **"bacterial count"** refers hereinafter to any quantitative determination of bacterial populations or index of bacterial growth and cell numbers (biomass). This includes, but is not limited to the two widely used methods for determining bacterial numbers, namely the standard, or viable, plate count method and spectrophotometric (turbidimetric) analysis. A non limiting example of plate count is the colony-forming units (CFUs) technique. Spectrophotometric (turbidimetric) analysis may refer to increased turbidity in a culture. By using a spectrophotometer, the amount of transmitted light decreases as the cell population increases. The transmitted light is converted to electrical energy, and this is indicated on a galvanometer. The reading, called absorbance or optical density, indirectly reflects the number of bacteria.

The term **"growth medium"** or **"growth media"** refers hereinafter to water supplemented with components such as, but not limited to nitrogen, phosphorus, potassium, calcium, iron, zinc, copper, manganese, magnesium, urea, nitrites, nitrates, ammonia, sugars (such as dextrose, glucose, lactose) concentration in the range of about 0.01-3.0 % w/v, amino acids and/or peptides and/or vitamins sources (such as yeast extract, enzymatic digest of casein, enzymatic digest of gelatin) in a concentration range of about 0.01-3.0 % w/v thereof, amino acids or mixes thereof (such as L-ArginineoHCl, L-Cysteine, L-Glutamine, Glycine, L-HistidineoHCloH2O, L-Isoleucine, L-Leucine, L-LysineoHCl, L-Methionine, L-Phenylalanine, L-Serine, L-Threonine, L-Tryptophan, L-Tyrosine 2Nao2H2O, L-Valine) in a concentration range of about 0.0001- 0.3 g/l, microelements (such as Choline Chloride, Folic Acid, myo-Inositol, Niacinamide, D-Pantothenic Acid hemicalcium, Calcium Chloride, Ferric Nitrate, Magnesium Sulfate, Potassium Chloride, Sodium Chloride, Sodium Phosphate) in a concentration range of about 0.0001- 0.3 g/l, vitamins, (NH₄)₂SO₄; Ca(NO₃)₂.4H2O; CaCl₂.6H2O; COCl₂.6H2O; CoSO₄.7H2O; CuCl₂.2H2O; CuSO₄.5H2O; FeCitrat; FeCl₃.6H20; FeSO₄.7H2O; FeTartrat; H2MoO₄.4H2O; H₃BO₃; H₃PO₄; KI; K₂HPO₄; K₂SO₄; KCl ;KH₂PO₄; KNO₃; Mg(NO₃)₂; MgSO₄.7H2O; MnCl₂.4H2O; MnSO₄.H2O ; Na₂MoO₄.2H2O; NaCl; NH₄H₂PO₄; NH₄NO₃; ZnSO₄.7H2O; inorganic (NH₄)₂SO₄; Ca(NO₃)₂.4H2O; CaCl₂.6H2O; H₃BO₃; H₃PO₄; KCl; K₂SO₄; K₂CO₃; MgSO₄.7H2O; Na₂MoO₄.2H2O; NaCl; NaHCO3; Fe-EDTA; Zn-EDTA; Cu-EDTA, manure; urea; Na2EDTA; Hutner ; Hoagland-A ; Hoagland-B ; Pirson ;Hoagland-C ; Steinberg ; Schenk and Hildebrandt, Murashige, and any combination thereof.

According to some embodiments, the growth media is controlled and manipulated to achieve predetermined conditions and parameters such as temperature range of the growth media, temperature range of the atmosphere, water content and treatment procedure (such as de-nitrification, mechanical, filtration), illumination intensity, aeration, oxygen concentration, pH and any combination thereof.

The term **"Dietary Reference Intake"** or **"DRI"** or **"recommended daily intake"** or "RDI" refers hereinafter to any standard which pertains to the daily intake level of a nutrient that is considered to be sufficient to meet the requirements to sustain healthy individuals. RDI standards may include, in a non-limiting example, Recommended Dietary Allowance (RDA), Estimated Average Requirement (EAR), Adequate Intake (AI) and Upper Intake Level (UL) standards.

It is herein acknowledged that the vitamin B12 dietary reference intake ranges from 0.4 to about 3 µg per day. In specific embodiments, the vitamin B12 dietary reference intake for an adult ranges from about 2 to about 3 µg per day according to the US health authorities, and about 1.5 µg per day according to the UK health authorities. According to a relatively new study, the DRI should be about 4 to about 7 µg per day.

Reference is now made to Table 1, listing the current RDAs for vitamin B12 in micrograms (mcg). For infants aged 0 to 12 months, the Food and Nutrition Board (FNB) at the Institute of Medicine (IOM) of the National Academies (formerly National Academy of Sciences) established an Adequate Intake (AI) for vitamin B12 that is equivalent to the mean intake of vitamin B12 in healthy, breastfed infants.

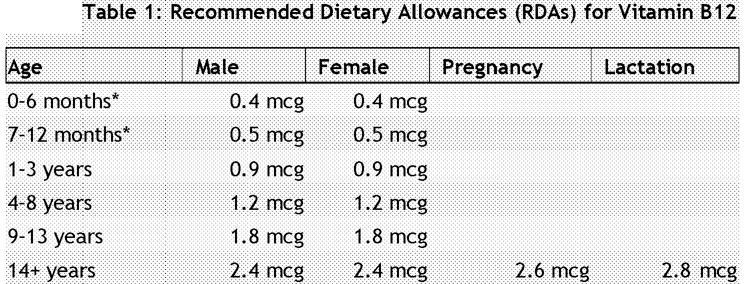

It is according to one embodiment of the present invention to disclose a method for producing vitamin B12 enriched Duckweed Bacterial Culture (DBC) composition, wherein the method comprises steps of: (a) inoculating at least one Lemnoideae species and at least one vitamin B12 producing bacteria species in a volume of growth media; (b) incubating the at least one Lemnoideae species and the at least one vitamin B12 producing bacteria species under predetermined conditions to provide a Duckweed- Bacterial Culture (DBC); (c) plotting Lemnoideae plant biomass at the predetermined conditions against time; (d) plotting bacterial count of the at least one B12 producing bacteria species at the predetermined conditions against time; (e) plotting vitamin B12 content in the DBC at the predetermined conditions against time; (f) determining time intervals within the plots characterized by DBC with highest vitamin B12 content; and (g) harvesting the DBC at the predetermined time intervals, thereby providing vitamin B12 enriched DBC composition.

It is further within the scope to disclose the method as defined in any of the above, wherein the step of incubating additionally comprises steps of selecting the predetermined conditions designed for (i) optimal growth of the at least one Lemnoideae species plant, and (ii) optimal fermentation of the vitamin B12 synthesizing bacteria associated therewith.

It is further within the scope to disclose the method as defined in any of the above, wherein the step of incubating additionally comprises steps of growing the at least one Lemnoideae species and the at least one vitamin B12 producing bacteria species under predetermined conditions to provide association between the at least one bacterial species and the at least one Lemnoideae species.

It is further within the scope to disclose the method as defined in any of the above, additionally comprises steps of selecting the association from the group consisting of: symbiotic interaction, persistent mutualism, persistent biological interaction, mutualism, interspecies reciprocal altruism, commensalistic interaction, parasitic symbiosis, obligate interaction, facultative interaction, obligate for both species, obligate for one but facultative for the other, facultative for both species, ectosymbiosis, endosymbiosis, commensal ectosymbiosis, mutualist ectosymbiosis, ectoparasitism, conjunctive symbiosis, disjunctive symbiosis, antagonistic or antipathetic symbiosis or relationship, necrotrophic interaction, biotrophic interaction, amensalism, competition relationship, antibiosis relationship, synnecrosis and any combination thereof.

It is further within the scope to disclose the method as defined in any of the above, wherein the step of inoculating comprises steps of growing the at least one Lemnoideae species and the at least one B12 producing bacteria species as a batch or as a continuous culture.

It is further within the scope to disclose the method as defined in any of the above, wherein the step of inoculating comprises steps of selecting the volume of growth media from the group consisting of: an aqueous plant growing facility and a microbial growing facility.

It is further within the scope to disclose the method as defined in any of the above, wherein the step of inoculating comprises steps of selecting the volume of growth media from the group consisting of: a pool, a channel, an aquarium, a fermenter, a bioreactor, cobbles and any other substrate.

It is further within the scope to disclose the method as defined in any of the above, wherein the step of incubating additionally comprising steps of selecting the predetermined conditions for growth of the Lemnoideae species biomass from the group consisting of: mineral composition of the growth media, mineral concentration of the growing media, urea concentration, nitrites and nitrates concentration, total ammonia concentration, temperature range of the growing media, temperature range of the atmosphere, water treatment procedure, illumination intensity, aeration, oxygen concentration, pH and any combination thereof.

It is further within the scope to disclose the method as defined in any of the above, additionally comprising steps of selecting the mineral from the group consisting of: Mg²⁺, Mn, Zn, Fe²⁺, Zn²⁺, Mn²⁺, CaCl₂ and any combination thereof.

It is further within the scope to disclose the method as defined in any of the above, additionally comprising steps of selecting the water treatment procedure from the group consisting of: de-nitrification, mechanical, filtration and any combination thereof.

It is further within the scope to disclose the method as defined in any of the above, wherein the step of incubating additionally comprising steps of selecting the predetermined conditions for symbiotic fermentation and vitamin B12 synthesis of the bacterial species from the group consisting of: sugar concentration in the range of about 0.01-3.0 % w/v, amino acids and/or peptides and/or vitamins sources in a concentration range of about 0.01-3.0 % w/v thereof, amino acids or mixes thereof in a concentration range of about 0.0001- 0.3 g/l, microelements in a concentration range of about 0.0001- 0.3 g/l, vitamins and any combination thereof.

It is further within the scope to disclose the method as defined in any of the above, additionally comprising steps of selecting the sugar from the group consisting of dextrose, glucose, lactose and any combination thereof.

It is further within the scope to disclose the method as defined in any of the above, additionally comprising steps of selecting the amino acids and/or peptides and/or vitamins sources from the group consisting of Yeast extract, Enzymatic Digest of Casein, Enzymatic Digest of Gelatin and any combination thereof.

It is further within the scope to disclose the method as defined in any of the above, additionally comprising steps of selecting the amino acids or mixes thereof from the group consisting of L-ArginineoHCl, L-Cysteine, L-Glutamine, Glycine, L-HistidineoHCloH₂O, L-Isoleucine, L-Leucine, L-LysineoHCl, L-Methionine, L-Phenylalanine, L-Serine, L-Threonine, L-Tryptophan, L-Tyrosine 2Nao2H2O, L-Valine and any combination thereof.

It is further within the scope to disclose the method as defined in any of the above, additionally comprising steps of selecting the microelements from the group consisting of: Choline Chloride, Folic Acid, myo-Inositol, Niacinamide, D-Pantothenic Acid hemicalcium, Calcium Chloride, Ferric Nitrate, Magnesium Sulfate, Potassium Chloride, Sodium Chloride, Sodium Phosphate and any combination thereof.

It is further within the scope to disclose the method as defined in any of the above, additionally comprising steps of selecting the bacteria from the group consisting of: *Pseudomonas species such as P. aeruginosa, P. florescenza, P. murina, Bacillus species, Methanobacterium species, Propionibacterium species, Acetobacterium, Aerobacter, Agrobacterium, Alcaligenes, Azotobacter, Clostridium, Flavobacterium, Lactobacillus, Micromonospora, Mycobacterium, Nocardia, Propionibacterium, Protaminobacter, Proteus, Pseudomonas, Rhizobium, Salmonella, Serratia, Streptomyces, Streptococcus, Xanthomonas* and any combination thereof.

It is further within the scope to disclose the method as defined in any of the above, wherein the step of determining additionally comprises steps of determining the growth curves of the at least one Lemnoideae species and the at least one B12 producing bacteria species against time and identifying time intervals characterized by highest biomass of the at least one Lemnoideae species and highest bacterial cell count of the at least one bacteria species.

It is further within the scope to disclose the method as defined in any of the above, additionally comprising steps of selecting the biomass from the group consisting of: whole Lemnoideae biomass, fresh Lemnoideae biomass, dry Lemnoideae biomass and any combination thereof.

It is further within the scope to disclose the method as defined in any of the above additionally comprising steps of selecting the at least one Lemnoideae species from a species belonging to the genera group consisting of: *Landoltia, Lemna, Spirodela, Wolffia, Wolffiella* and any combination thereof.

It is further within the scope to disclose the method as defined in any of the above, additionally comprising steps of selecting the at least one Lemnoideae species from the group consisting of: *Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta* and any combination thereof.

It is further within the scope to disclose the method as defined in any of the above, additionally comprises steps of providing vitamin B12 enriched DBC composition comprising between about 0.01 and about 100 µg vitamin B12 per 100g of the DBC.

The present invention further provides a composition comprising a vitamin B12 enriched Duckweed- Bacterial Culture (DBC), wherein the composition comprises at least one Lemnoideae species and at least one B12 producing bacteria species, further wherein the vitamin B12 content in the composition is in the range of between about 0.01 µg and about 100 µg per 100g of the DBC.

It is according to one embodiment to disclose the composition as defined in any of the above, wherein the vitamin B12 content in the composition is a predetermined percentage of at least 20% of the vitamin B12 recommended Daily Value (DV).

It is according to another embodiment to disclose the composition as defined in any of the above, wherein the vitamin B12 content in the composition complies with the vitamin B12 recommended daily intake (RDI) standard for an adult ranging from about 0.4 µg to about 3 µg per day.

It is according to another embodiment to disclose the composition as defined in any of the above, wherein the at least one B12 producing bacteria species is in association with the at least one Lemnoideae species.

It is according to another embodiment to disclose the composition as defined in any of the above, wherein the association is selected from the group consisting of: symbiotic interaction, persistent mutualism, persistent biological interaction, mutualism, interspecies reciprocal altruism, commensalistic interaction, parasitic symbiosis, obligate interaction, facultative interaction, obligate for both species, obligate for one but facultative for the other, facultative for both species, ectosymbiosis, endosymbiosis, commensal ectosymbiosis, mutualist ectosymbiosis, ectoparasitism, conjunctive symbiosis, disjunctive symbiosis, antagonistic or antipathetic symbiosis or relationship, necrotrophic interaction, biotrophic interaction, amensalism, competition relationship, antibiosis relationship, synnecrosis and any combination thereof.

It is according to another embodiment to disclose the composition as defined in any of the above, wherein the B12 producing bacteria are selected from the group consisting of: *Pseudomonas species such as P. aeruginosa, P. florescenza, P. murina, Bacillus species, Methanobacterium species, Propionibacterium species, Acetobacterium, Aerobacter, Agrobacterium, Alcaligenes, Azotobacter, Clostridium, Flavobacterium, Lactobacillus, Micromonospora, Mycobacterium, Nocardia, Propionibacterium, Protaminobacter, Proteus, Pseudomonas, Rhizobium, Salmonella, Serratia, Streptomyces, Streptococcus, Xanthomonas* and any combination thereof.

It is according to another embodiment to disclose the composition as defined in any of the above, wherein the at least one Lemnoideae species containing biomass is selected from the group consisting of: whole Lemnoideae biomass, fresh Lemnoideae biomass, dry Lemnoideae biomass and any combination thereof.

It is according to another embodiment to disclose the composition as defined in any of the above, wherein the at least one Lemnoideae species belongs to a genera selected from the group consisting of: *Landoltia, Lemna, Spirodela, Wolffia, Wolffiella* and any combination thereof.

It is according to another embodiment to disclose the composition as defined in any of the above, wherein the at least one Lemnoideae species is selected from the group consisting of: *Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta* and any combination thereof.

It is further within the scope to disclose a composition comprising vitamin B12 enriched Duckweed Bacterial Culture (DBC) produced by the method as defined in any of the above.

It is further within the scope to disclose the composition produced by the method as defined in any of the above, wherein the predetermined conditions are selected from the group consisting of conditions for growth of the Lemnoideae species biomass, conditions for fermentation and vitamin B12 synthesis of the bacterial species and a combination thereof.

It is further within the scope to disclose the composition produced by the method as defined in any of the above, wherein the volume of growth media is a batch or a continuous culture.

It is further within the scope to disclose the composition produced by the method as defined in any of the above, wherein the volume of growth media is selected from the group consisting of: an aqueous plant growing facility and a microbial growing facility.

It is further within the scope to disclose the composition produced by the method as defined in any of the above, wherein the volume of growth media is selected from the group consisting of: a pool, a channel, an aquarium, a fermenter, a bioreactor, cobbles and any other substrate.

It is further within the scope to disclose the composition produced by the method as defined in any of the above, wherein the predetermined conditions for growth of the Lemnoideae species biomass are selected from the group consisting of: mineral composition of the growing media, mineral concentration of the growing media, urea concentration, nitrites and nitrates concentration, total ammonia concentration, temperature range of the growing media, temperature range of the atmosphere, water treatment procedure, illumination intensity, aeration, oxygen concentration, pH and any combination thereof.

It is further within the scope to disclose the composition produced by the method as defined in any of the above, wherein the mineral is selected from the group consisting of: Mg²⁺, Mn, Zn, Fe²⁺, Zn²⁺, Mn²⁺, CaCl₂ and any combination thereof.

It is further within the scope to disclose the composition produced by the method as defined in any of the above, wherein the water treatment procedure is selected from the group consisting of: de-nitrification, mechanical, filtration and any combination thereof.

It is further within the scope to disclose the composition produced by the method as defined in any of the above, wherein the predetermined conditions for fermentation and vitamin B12 synthesis of the bacterial species are selected from the group consisting of: sugar concentration in the range of about 0.01-3.0 % w/v, amino acids and/or peptides and/or vitamins sources in a concentration range of about 0.01-3.0 % w/v thereof, amino acids or mixes thereof in a concentration range of about 0.0001- 0.3 g/l, microelements in a concentration range of about 0.0001- 0.3 g/l, vitamins and any combination thereof.

It is further within the scope to disclose the composition produced by the method as defined in any of the above, wherein the sugars are selected from the group consisting of dextrose, glucose, lactose and any combination thereof.

It is further within the scope to disclose the composition produced by the method as defined in any of the above, wherein the amino acids and/or peptides and/or vitamins sources are selected from the group consisting of Yeast extract, Enzymatic Digest of Casein, Enzymatic Digest of Gelatin and any combination thereof.

It is further within the scope to disclose the composition produced by the method as defined in any of the above, wherein the amino acids or mixes thereof are selected from the group consisting of L-ArginineoHCl, L-Cysteine, L-Glutamine, Glycine, L-HistidineoHCloH2O, L-Isoleucine, L-Leucine, L-LysineoHCl, L-Methionine, L-Phenylalanine, L-Serine, L-Threonine, L-Tryptophan, L-Tyrosine 2Nao2H2O, L-Valine and any combination thereof.

It is further within the scope to disclose the composition produced by the method as defined in any of the above, wherein the microelements are selected from the group consisting of: Choline Chloride, Folic Acid, myo-Inositol, Niacinamide, D-Pantothenic Acid hemicalcium, Calcium Chloride, Ferric Nitrate, Magnesium Sulfate, Potassium Chloride, Sodium Chloride, Sodium Phosphate and any combination thereof.

It is further within the scope of the present invention to disclose a system for producing vitamin B12 enriched Duckweed Bacterial Culture (DBC) composition, wherein the system comprises: (a) at least one inoculum of Lemnoideae species and at least one inoculum of vitamin B12 producing bacteria species for cultivation in a volume of growth media; (b) an incubation means for incubating the at least one Lemnoideae species inoculum and the at least one vitamin B12 producing bacteria species inoculum under predetermined conditions to provide a Duckweed- Bacterial Culture (DBC); (c) means for determining time intervals within the plots characterized by DBC with highest vitamin B12 content; and (d) means for harvesting the DBC at the predetermined time intervals, thereby providing vitamin B12 enriched DBC composition.

It is further within the scope of the present invention to disclose the system as defined above, wherein the system additionally comprises at least one plotting means selected from the group consisting of: (a) plotting means for plotting Lemnoideae plant biomass at the predetermined conditions against time; (b) plotting means for plotting bacterial count of the at least one B12 producing bacteria species at the predetermined conditions against time; and (c) plotting means for plotting vitamin B12 content in the DBC at the predetermined conditions against time.

It is further within the scope of the present invention to disclose a composition comprising a vitamin B12 enriched extract of at least one Lemnoideae species and at least one B12 producing bacteria species, further wherein the vitamin B12 content in the composition is in the range of between about 0.01 and about 100 µg per 100g of the DBC.

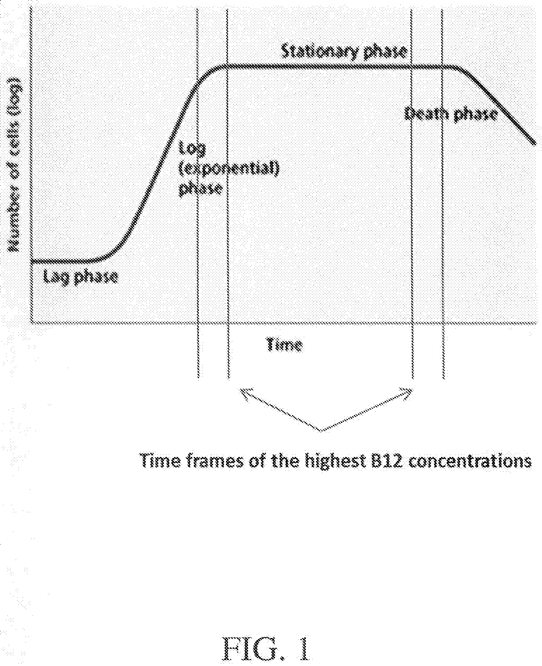

Reference is now made to **Fig. 1****,** illustrating a typical bacterial growth curve or kinetic curve as known in the prior art. This curve generally describes the phases of bacterial growth versus time, after inoculating the bacteria into a selected growth medium. As can be seen, the first stage in the growth curve is a period of adaptation, called the **lag phase.** During the lag phase, bacteria adapt themselves to growth conditions. It is the period where the individual bacteria are maturing and not yet able to divide. During the lag phase of the bacterial growth cycle, synthesis of RNA, enzymes and other molecules occurs.

Following the lag phase, the rate of growth of the organism steadily increases; this period is the **log or exponential phase.** The log phase is a period characterized by cell duplication. The duplication rate is proportional to the particular population. If growth is not limited, doubling will continue at a constant rate so both the number of cells and the rate of population increase doubles with each consecutive time period. For this type of exponential growth, plotting the natural logarithm of cell number against time, produces a straight line. The slope of this line is the specific growth rate of the organism, which is a measure of the number of divisions per cell per unit time. The actual rate of this growth (i.e. the slope of the line in the figure) depends upon the growth conditions, which affect the frequency of cell division events and the probability of both daughter cells surviving. Exponential growth cannot continue indefinitely, because the medium is depleted of nutrients and enriched with wastes.

Thus, after a certain time of exponential phase, the rate of growth slows down, due to the continuously falling concentrations of nutrients and/or continuously increasing (accumulating) concentrations of toxic substances. This phase, where the growth increase ceases is called a **stationary phase** or a steady state. The biomass remains constant, except when certain accumulated chemicals in the culture lyse the cells (chemolysis). Thus, the stationary phase is often due to a growth-limiting factor such as the depletion of an essential nutrient, and/or the formation of an inhibitory product such as an organic acid. Stationary phase results from a situation in which growth rate and death rate are equal. The number of new cells created is limited by the growth factor and as a result the rate of cell growth matches the rate of cell death. The result is a horizontal linear part of the curve during the stationary phase.

Unless other micro-organisms contaminate the culture, the chemical constitution remains unchanged. When all of the nutrients in the medium are consumed, or if the concentration of toxins is too high, the cells may become senescent and begin to die. In this **death phase,** the total amount of biomass may not decrease, but the number of viable organisms will decrease. At death phase, (Decline phase) bacteria die. This could be due to lack of nutrients, a temperature which is too high or low etc.

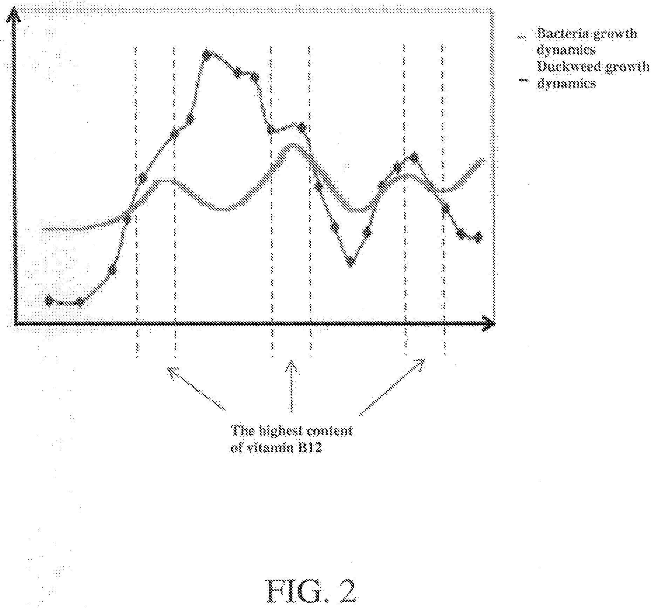

It is according to a main aspect of the invention that time intervals of high bacterial vitamin B12 concentration have been identified in accordance with the bacterial growth curve exemplified in **Fig. 1****.** It can be seen that a time period of higher vitamin B12 concentration is identified at the interval between the exponential phase and the stationary phase, where the growth rate slows, due to the continuously falling concentrations of nutrients and/or continuously increasing (accumulating) concentrations of toxic substances. At this interval, the increase of the growth rate is checked. Another period or time interval of higher vitamin B12 concentration is identified between the end of the stationary phase and before the beginning of the death phase.
Reference is now made to **Fig. 2****,** illustrating an exemplified duckweed-bacteria culture (DBC) growth curve or profile, as an embodiment of the present invention. It is within the scope of the present invention that at least one Lemnoideae species and at least one vitamin B12 producing bacteria species are grown under predetermined conditions for (i) maximal effective growth of said Lemnoideae species biomass, and (ii) maximal and effective fermentation and vitamin B12 synthesis of said bacterial species. As shown in this figure, time intervals or time frames of maximal B12 production and duckweed biomass are identified. These time intervals are characterized by maximal DBC B12 production. In other words, at these time intervals, DBC with enriched vitamin B12 concentration is harvested. This highly nutritional vitamin B12 source may be used as fresh or dry material characterized by high concentrations of natural vitamin B12 for human consumption.

In some embodiments of the invention, the vitamin B12 content in said DBC is a predetermined percentage of the vitamin B12 Dietary Reference Intakes (DRIs) standard.

In other embodiments of the invention, the vitamin B12 content in said DBC is a predetermined percentage of the Daily Value (DV) of vitamin B12.

It is herein acknowledged that intake recommendations for vitamin B12 and other nutrients are provided in the Dietary Reference Intakes (DRIs) developed by the Food and Nutrition Board (FNB) at the Institute of Medicine (IOM) of the National Academies. The term 'DRI' is herein generally refers to a set of reference values used for planning and assessing nutrient intakes of healthy people. These values, which vary by age and gender, include:
Recommended Dietary Allowance (RDA), defined as the average daily level of intake sufficient to meet the nutrient requirements of nearly all (97%-98%) healthy individuals.

Adequate Intake (AI) established when evidence is insufficient to develop an RDA and is set at a level assumed to ensure nutritional adequacy.

Tolerable Upper Intake Level (UL), which is defined as the maximum daily intake unlikely to cause adverse health effects.

It is further acknowledged that the dietary reference intake (DRI) of vitamin B12 for an adult ranges from 0.4 to 3 µg per day in the US and 1.5 µg per day in the UK. But according to recent studies, the DRI should be between 4 to 7 µg per day. It is noted that the Center for Food Safety and Applied Nutrition recommends 6 µg per day, based on a caloric intake of 2,000 calories, for adults and children four or more years of age.

It is further noted that vitamin B12 is believed to be safe when used orally in amounts that do not exceed the recommended dietary allowance (RDA).

According to some aspects, the vitamin B12 recommended dietary amounts (RDAs) are 2.4 micrograms daily for ages 14 years and older, 2.6 micrograms daily for pregnant females, and 2.8 micrograms daily for breastfeeding females. Adults over 50 years of age should meet the RDA by eating foods reinforced with B12 or by taking a vitamin B12 supplement. It was reported that supplementation of 25-100 micrograms daily has been used to maintain vitamin B12 levels in older people.

Reference is now made to Daily Value (DV) of vitamin B12. It is herein acknowledged that DVs were developed by the U.S. Food and Drug Administration (FDA) to help consumers determine the level of various nutrients in a standard serving of food in relation to their approximate requirement for it. The DV for vitamin B12 is 6.0 mcg. However, the FDA does not require food labels to list vitamin B12 content unless a food has been fortified with this nutrient. Foods providing 20% or more of the DV are considered to be high or enriched sources of a nutrient, but foods providing lower percentages of the DV also contribute to a healthful diet.

The present invention provides means and methods for producing a vitamin B12 enriched duckweed biomass composition comprising at least one Lemnoideae species and at least one vitamin B12 producing bacteria species, which produce Duckweed- Bacterial Culture (DBC) in predesigned conditions. The vitamin B12 enriched composition can be consumed by intake of a predetermined dosage of said composition, one or more times per day so as to meet the recommended vitamin B12 Dietary Reference Intake (DRI) of between about 0.4 mcg and about 3 mcg per day.

According to certain aspects, the composition of the present invention provides 20% or more of the vitamin B12 DV and thus is considered to be high or enriched source of this important nutrient.

According to other aspects, the composition of the present invention comprises between about 0.01 and about 100 µg vitamin B12 per 100g of said DBC.

As discussed above, it is within the scope that the vitamin B12 enriched composition is preferably provided as dry material to achieve high concentrations of vitamin B12 in a single dose of the composition.

## Claims

1. A method for producing vitamin B12 enriched Duckweed Bacterial Culture (DBC) composition, wherein said method comprises steps of:
a. inoculating at least one Lemnoideae species and at least one vitamin B12 producing bacteria species in a volume of growth media;
b. incubating said at least one Lemnoideae species and said at least one vitamin B12 producing bacteria species under predetermined conditions to provide a Duckweed-Bacterial Culture (DBC);
c. plotting Lemnoideae plant biomass at said predetermined conditions against time;
d. plotting bacterial count of said at least one B12 producing bacteria species at said predetermined conditions against time;
e. plotting vitamin B12 content in said DBC at said predetermined conditions against time;
f. determining time intervals within said plots **characterized by** DBC with highest vitamin B12 content; and
g. harvesting said DBC at said predetermined time intervals, thereby providing vitamin B12 enriched DBC composition.

2. The method according to claim **1,** wherein said step of incubating additionally comprises at least one step of:
a. selecting said predetermined conditions designed for (i) optimal growth of said at least one Lemnoideae species plant, and (ii) optimal fermentation of said vitamin B12 synthesizing bacteria associated therewith;
b. growing said at least one Lemnoideae species and said at least one vitamin B12 producing bacteria species under predetermined conditions to provide association between said at least one bacterial species and said at least one Lemnoideae species;
c. selecting said predetermined conditions for growth of said Lemnoideae species biomass from the group consisting of: mineral composition of the growth media, mineral concentration of the growing media, urea concentration, nitrites and nitrates concentration, total ammonia concentration, temperature range of the growing media, temperature range of the atmosphere, water treatment procedure, illumination intensity, aeration, oxygen concentration, pH and any combination thereof; and
d. selecting said predetermined conditions for symbiotic fermentation and vitamin B12 synthesis of said bacterial species from the group consisting of: sugar concentration in the range of 0.01-3.0 % w/v, amino acids and/or peptides and/or vitamins sources in a concentration range of 0.01-3.0 % w/v thereof, amino acids or mixes thereof in a concentration range of 0.0001 - 0.3 g/l, microelements in a concentration range of 0.0001 - 0.3 g/l, vitamins and any combination thereof.

3. The method according to claim **2,** additionally comprises at least one step of:
a. selecting said association from the group consisting of: symbiotic interaction, persistent mutualism, persistent biological interaction, mutualism, interspecies reciprocal altruism, commensalistic interaction, parasitic symbiosis, obligate interaction, facultative interaction, obligate for both species, obligate for one but facultative for the other, facultative for both species, ectosymbiosis, endosymbiosis, commensal ectosymbiosis, mutualist ectosymbiosis, ectoparasitism, conjunctive symbiosis, disjunctive symbiosis, antagonistic or antipathetic symbiosis or relationship, necrotrophic interaction, biotrophic interaction, amensalism, competition relationship, antibiosis relationship, synnecrosis and any combination thereof;
b. selecting said mineral from the group consisting of: Mg²⁺, Mn, Zn, Fe²⁺, Zn²⁺, Mn²⁺, CaCl₂ and any combination thereof;
c. selecting said water treatment procedure from the group consisting of: de-nitrification, mechanical, filtration and any combination thereof;
d. selecting said sugar from the group consisting of dextrose, glucose, lactose and any combination thereof;
e. selecting said amino acids and/or peptides and/or vitamins sources from the group consisting of Yeast extract, Enzymatic Digest of Casein, Enzymatic Digest of Gelatin and any combination thereof;
f. selecting said amino acids or mixes thereof from the group consisting of L-ArginineoHCl, L-Cysteine, L-Glutamine, Glycine, L-HistidineoHCloH2O, L-Isoleucine, L-Leucine, L-LysineoHCl, L-Methionine, L-Phenylalanine, L-Serine, L-Threonine, L-Tryptophan, L-Tyrosine 2Nao2H2O, L-Valine and any combination thereof; and
g. selecting said microelements from the group consisting of: Choline Chloride, Folic Acid, myo-Inositol, Niacinamide, D-Pantothenic Acid hemicalcium, Calcium Chloride, Ferric Nitrate, Magnesium Sulfate, Potassium Chloride, Sodium Chloride, Sodium Phosphate and any combination thereof.

4. The method according to claim **1,** wherein said step of inoculating comprises at least one step of:
a. growing said at least one Lemnoideae species and said at least one B 12 producing bacteria species as a batch or as a continuous culture;
b. selecting said volume of growth media from the group consisting of: an aqueous plant growing facility and a microbial growing facility; and
c. selecting said volume of growth media from the group consisting of: a pool, a channel, an aquarium, a fermenter, a bioreactor, cobbles and any other substrate.

5. The method according to claim **1,** additionally comprising at least one step of:
a. selecting said bacteria from the group consisting of: *Pseudomonas species, Bacillus species, Methanobacterium species, Propionibacterium species, Acetobacterium, Aerobacter, Agrobacterium, Alcaligenes, Azotobacter, Clostridium, Flavobacterium, Lactobacillus, Micromonospora, Mycobacterium, Nocardia, Protaminobacter, Proteus, Rhizobium, Salmonella, Serratia, Streptomyces, Streptococcus, Xanthomonas* and any combination thereof;
b. selecting said biomass from the group consisting of: whole Lemnoideae biomass, fresh Lemnoideae biomass, dry Lemnoideae biomass and any combination thereof;
c. selecting said at least one Lemnoideae species from a species belonging to the genera group consisting of: *Landoltia, Lemna, Spirodela, Wolffia, Wolffiella* and any combination thereof;
d. selecting said at least one Lemnoideae species from the group consisting of: *Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta* and any combination thereof; and
e. providing vitamin B12 enriched DBC composition comprising between 0.01 and 100 µg vitamin B12 per 100g of said DBC.

6. The method according to claim 1, wherein said step of determining additionally comprises steps of determining the growth curves of said at least one Lemnoideae species and said at least one B12 producing bacteria species against time and identifying time intervals **characterized by** highest biomass of said at least one Lemnoideae species and highest bacterial cell count of said at least one bacteria species.

7. A composition comprising a vitamin B12 enriched Duckweed- Bacterial Culture (DBC), wherein said composition comprises at least one Lemnoideae species and at least one B12 producing bacteria species, further wherein said vitamin B12 content in said composition is in the range of between 0.01 and 100 µg per 100g of said DBC.

8. The composition according to claim 7, wherein at least one of the following holds true:
a. said vitamin B12 content in said composition is a predetermined percentage of at least 20% of the vitamin B12 recommended Daily Value (DV);
b. said vitamin B12 content in said composition complies with the vitamin B12 recommended daily intake (RDI) standard for an adult ranging from 0.4 µg to 3 µg per day;
c. said at least one B12 producing bacteria species is in association with said at least one Lemnoideae species;
d. said Lemnoideae species and said B12 producing bacteria species are in an association, said association selected from the group consisting of: symbiotic interaction, persistent mutualism, persistent biological interaction, mutualism, interspecies reciprocal altruism, commensalistic interaction, parasitic symbiosis, obligate interaction, facultative interaction, obligate for both species, obligate for one but facultative for the other, facultative for both species, ectosymbiosis, endosymbiosis, commensal ectosymbiosis, mutualist ectosymbiosis, ectoparasitism, conjunctive symbiosis, disjunctive symbiosis, antagonistic or antipathetic symbiosis or relationship, necrotrophic interaction, biotrophic interaction, amensalism, competition relationship, antibiosis relationship, synnecrosis and any combination thereof;
e. said B12 producing bacteria species is selected from the group consisting of: *Pseudomonas species, Bacillus species, Methanobacterium species, Propionibacterium species, Acetobacterium, Aerobacter, Agrobacterium, Alcaligenes, Azotobacter, Clostridium, Flavobacterium, Lactobacillus, Micromonospora, Mycobacterium, Nocardia, Protaminobacter, Proteus, Rhizobium, Salmonella, Serratia, Streptomyces, Streptococcus, Xanthomonas* and any combination thereof;
f. said Lemnoideae species containing biomass is selected from the group consisting of: whole Lemnoideae biomass, fresh Lemnoideae biomass, dry Lemnoideae biomass and any combination thereof;
g. said Lemnoideae species belongs to a genera selected from the group consisting of: *Landoltia, Lemna, Spirodela, Wolffia, Wolffiella* and any combination thereof; and
h. said Lemnoideae species is selected from the group consisting of: *Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta* and any combination thereof.

9. A composition comprising vitamin B 12 enriched Duckweed Bacterial Culture (DBC) produced by the method according to claim **1.**

10. The composition according to claim **9,** wherein at least one of the following holds true:
a. said predetermined conditions are selected from the group consisting of conditions for growth of said Lemnoideae species biomass, conditions for fermentation and vitamin B 12 synthesis of said bacterial species and a combination thereof;
b. said volume of growth media is a batch or a continuous culture;
c. said volume of growth media is selected from the group consisting of: an aqueous plant growing facility and a microbial growing facility; and
d. said volume of growth media is selected from the group consisting of: a pool, a channel, an aquarium, a fermenter, a bioreactor, cobbles and any other substrate.

11. The composition according to claim **10,** wherein at least one of the following holds true:
a. said predetermined conditions for growth of said Lemnoideae species biomass are selected from the group consisting of: mineral composition of the growing media, mineral concentration of the growing media, urea concentration, nitrites and nitrates concentration, total ammonia concentration, temperature range of the growing media, temperature range of the atmosphere, water treatment procedure, illumination intensity, aeration, oxygen concentration, pH and any combination thereof; and
b. said predetermined conditions for fermentation and vitamin B12 synthesis of said bacterial species are selected from the group consisting of: sugar concentration in the range of 0.01-3.0 % w/v, amino acids and/or peptides and/or vitamins sources in a concentration range of 0.01-3.0 % w/v thereof, amino acids or mixes thereof in a concentration range of 0.0001 - 0.3 g/l, microelements in a concentration range of 0.0001 - 0.3 g/l, vitamins and any combination thereof.

12. The composition according to claim **11,** wherein at least one of the following holds true:
a. said mineral is selected from the group consisting of: Mg²⁺, Mn, Zn, Fe²⁺, Zn²⁺, Mn²⁺, CaCl₂ and any combination thereof;
b. said water treatment procedure is selected from the group consisting of: de-nitrification, mechanical, filtration and any combination thereof;
c. said sugars are selected from the group consisting of dextrose, glucose, lactose and any combination thereof;
d. said amino acids and/or peptides and/or vitamins sources are selected from the group consisting of Yeast extract, Enzymatic Digest of Casein, Enzymatic Digest of Gelatin and any combination thereof;
e. said amino acids or mixes thereof are selected from the group consisting of L-ArginineoHCl, L-Cysteine, L-Glutamine, Glycine, L-HistidineoHCloH2O, L-Isoleucine, L-Leucine, L-LysineoHCl, L-Methionine, L-Phenylalanine, L-Serine, L-Threonine, L-Tryptophan, L-Tyrosine 2Nao2H2O, L-Valine and any combination thereof; and
f. said microelements are selected from the group consisting of: Choline Chloride, Folic Acid, myo-Inositol, Niacinamide, D-Pantothenic Acid hemicalcium, Calcium Chloride, Ferric Nitrate, Magnesium Sulfate, Potassium Chloride, Sodium Chloride, Sodium Phosphate and any combination thereof.

13. A system for producing vitamin B12 enriched Duckweed Bacterial Culture (DBC) composition, wherein said system comprises:
a. at least one inoculum of Lemnoideae species and at least one inoculum of vitamin B12 producing bacteria species for cultivation in a volume of growth media;
b. an incubation means for incubating said at least one Lemnoideae species inoculum and said at least one vitamin B12 producing bacteria species inoculum under predetermined conditions to provide a Duckweed- Bacterial Culture (DBC);
c. means for determining time intervals within said plots **characterized by** DBC with highest vitamin B12 content; and
d. means for harvesting said DBC at said predetermined time intervals, thereby providing vitamin B12 enriched DBC composition.

14. The system according to claim **13,** wherein said system additionally comprises at least one plotting means selected from the group consisting of:
a. plotting means for plotting Lemnoideae plant biomass at said predetermined conditions against time;
b. plotting means for plotting bacterial count of said at least one B12 producing bacteria species at said predetermined conditions against time; and
c. plotting means for plotting vitamin B12 content in said DBC at said predetermined conditions against time.

15. A composition comprising a vitamin B12 enriched extract of at least one Lemnoideae species and at least one B12 producing bacteria species, further wherein said vitamin B12 content in said composition is in the range of between 0.01 and 100 µg per 100g of Duckweed Bacterial Culture (DBC).

## Patentansprüche

1. Verfahren zum Herstellen einer mit Vitamin B12 angereicherten Wasserlinsen-Bakterienkultur(Duckweed Bacterial Culture - DBC)-Zusammensetzung, wobei das Verfahren die folgenden Schritte umfasst:
a. Beimpfen eines Volumens von Nährmedien mit mindestens einer Lemnoideae-Art und mindestens einer Vitamin B12 produzierenden Bakterienart;
b. Inkubieren der mindestens einen Lemnoideae-Art und der mindestens einen Vitamin B12 produzierenden Bakterienart unter vorbestimmten Bedingungen, um eine Wasserlinsen-Bakterienkultur (Duckweed Bacterial Culture - DBC) bereitzustellen;
c. graphische Darstellung der Lemnoideae-Pflanzenbiomasse unter den vorbestimmten Bedingungen über die Zeit;
d. graphische Darstellung der Bakterienzahl der mindestens einen B12 produzierenden Bakterienart unter den vorbestimmten Bedingungen über die Zeit;
e. graphische Darstellung des Vitamin B12-Gehalts in der DBC unter den vorbestimmten Bedingungen über die Zeit;
f. Bestimmen von Zeitintervallen in den graphischen Darstellungen, die durch DBC mit dem höchsten Vitamin B12-Gehalt gekennzeichnet sind; und
g. Ernten der DBC bei den vorbestimmten Zeitintervallen, wodurch eine mit Vitamin B12 angereicherte DBC-Zusammensetzung bereitgestellt wird.

2. Verfahren nach Anspruch 1, wobei der Schritt des Inkubierens zusätzlich mindestens einen der folgenden Schritte umfasst:
a. Auswählen der vorbestimmten Bedingungen, die für (i) optimales Wachstum der mindestens einen Pflanze der Lemnoideae-Art und (ii) optimale Düngung der damit verbundenen Vitamin B12 synthetisierenden Bakterien konzipiert sind;
b. Züchten der mindestens einen Lemnoideae-Art und der mindestens einen Vitamin B12 produzierenden Bakterienart unter vorbestimmten Bedingungen, um eine Verbindung zwischen der mindestens einen Bakterienart und der mindestens einen Lemnoideae-Art bereitzustellen;
c. Auswählen der vorbestimmten Bedingungen zum Wachstum der Biomasse der Lemnoideae-Art aus der Gruppe bestehend aus: mineralischer Zusammensetzung der Nährmedien, mineralischer Konzentration der Nährmedien, Harnstoffkonzentration, Nitrit- und Nitratkonzentration, Gesamtammoniakkonzentration, Temperaturbereich der Züchtungsmedien, Temperaturbereich der Atmosphäre, Wasserbehandlungsverfahren, Beleuchtungsstärke, Belüftung, Sauerstoffkonzentration, pH-Wert und jeder Kombination davon; und
d. Auswählen der vorbestimmten Bedingungen für eine symbiotische Fermentation und eine Vitamin B12-Synthese der Bakterienart aus der Gruppe bestehend aus: Zuckerkonzentration im Bereich von 0,01-3,0 % w/v, Aminosäuren und/oder Peptide und/oder Vitaminquellen in einem Konzentrationsbereich von 0,01-3,0 % w/v davon, Aminosäuren oder Mischungen davon in einem Konzentrationsbereich von 0,0001-0,3 g/l, Spurenelementen in einem Konzentrationsbereich von 0,0001-0,3 g/l, Vitaminen und jeder Kombination davon.

3. Verfahren nach Anspruch 2, das zusätzlich mindestens einen der folgenden Schritte umfasst:
a. Auswählen der Verbindung aus der Gruppe bestehend aus: symbiotischer Wechselbeziehung, persistentem Mutualismus, persistenter biologischer Wechselbeziehung, Mutualismus, gegenseitigem Altruismus zwischen den Arten, kommensalistischer Wechselbeziehung, parasitischer Symbiose, obligater Wechselbeziehung, fakultativer Wechselbeziehung, obligatorisch für beide Arten, obligatorisch für eine, aber fakultativ für die andere, fakultativ für beide Arten, Ektosymbiose, Endosymbiose, kommensalistischer Ektosymbiose, mutualistischer Ektosymbiose, Ektoparasitismus, verbundener Symbiose, nicht verbundener Symbiose, antagonistischer oder antipathischer Symbiose oder Beziehung, nekrotrophischer Wechselbeziehung, biotrophischer Wechselbeziehung, Amensalismus, Konkurrenzbeziehung, antibiotischer Beziehung, Synnekrose und jeder Kombination davon;
b. Auswählen des Minerals aus der Gruppe bestehend aus: Mg²⁺, Mn, Zn, Fe²⁺, Zn²⁺, Mn²⁺, CaCl₂ und jeder Kombination davon;
c. Auswählen des Wasserbehandlungsverfahrens aus der Gruppe bestehend aus: Denitrifikation, mechanisch, Filtration und jeder Kombination davon;
d. Auswählen des Zuckers aus der Gruppe bestehend aus Dextrose, Glucose, Lactose und jeder Kombination davon;
e. Auswählen der Aminosäuren und/oder Peptide und/oder Vitaminquellen aus der Gruppe bestehend aus Hefeextrakt, enzymatischem Abbau von Casein, enzymatischem Abbau von Gelatine und jeder Kombination davon;
f. Auswählen der Aminosäuren oder deren Mischungen aus der Gruppe bestehend aus L-Arginin:HCl, L-Cystein, L-Glutamin, Glycin, L-Histidin:HCl:H₂O, L-Isoleucin, L-Leucin, L-Lysin:HCl, L-Methionin, L-Phenylalanin, L-Serin, L-Threonin, L-Tryptophan, L-Tyrosin:2Na:2H₂O, L-Valin und jeder Kombination davon; und
g. Auswählen der Spurenelemente aus der Gruppe bestehend aus: Cholinchlorid, Folsäure, Myo-Inositol, Niacinamid, D-Calcium-Pantothenat, Calciumchlorid, Eisen(III)Nitrat, Magnesiumsulfat, Kaliumchlorid, Natriumchlorid, Natriumphosphat und jeder Kombination davon.

4. Verfahren nach Anspruch 1, wobei der Schritt des Beimpfens mindestens einen der folgenden Schritte umfasst:
a. Züchten der mindestens einen Lemnoideae-Art und der mindestens einen B12 produzierenden Bakterienart als Batch- oder als kontinuierliche Kultur;
b. Auswählen des Volumens von Nährmedien aus der Gruppe bestehend aus: einer Wasserpflanzen-Zuchtanlage und einer Mikroben-Zuchtanlage; und
c. Auswählen des Volumens von Nährmedien aus der Gruppe bestehend aus: einem Teich, einer Rinne, einem Aquarium, einem Fermenter, einem Bioreaktor, Kieseln und jedem anderen Substrat.

5. Verfahren nach Anspruch 1, zusätzlich umfassend mindestens einen aus folgenden Schritten:
a. Auswählen der Bakterien aus der Gruppe bestehend aus folgenden Arten: *Pseudomonas, Bacillus, Methanobacterium, Propionibacterium, Acetobacterium, Aerobacter, Agrobacterium, Alcaligenes, Azotobacter, Clostridium, Flavobacterium, Lactobacillus, Micromonospora, Mycobacterium, Nocardia, Protaminobacter, Proteus, Rhizobium, Salmonella, Serratia, Streptomyces, Streptococcus, Xanthomonas* und jeder Kombination davon;
b. Auswählen der Biomasse aus der Gruppe bestehend aus: Biomasse aus ganzen Lemnoideae, Biomasse aus frischen Lemnoideae, Biomasse aus trockenen Lemnoideae und jeder Kombination davon;
c. Auswählen der mindestens einen Lemnoideae-Art aus einer Art, die zur Gruppe bestehend aus folgenden Gattungen gehört: *Landoltia, Lemna, Spirodela, Wolffia, Wolffiella* und jeder Kombination davon;
d. Auswählen der mindestens einen Lemnoideae-Art aus der Gruppe bestehend aus: *Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta* und jeder Kombination davon; und
e. Bereitstellen einer mit Vitamin B12 angereicherten DBC-Zusammensetzung, die zwischen 0,01 und 100 µg Vitamin B12 pro 100 g der DBC umfasst.

6. Verfahren nach Anspruch 1, wobei der Schritt des Bestimmens außerdem die Schritte des Bestimmens der Wachstumskurven der mindestens einen Lemnoideae-Art und der mindestens einen B12-produzierenden Bakterienart über die Zeit sowie des Identifizierens von Zeitintervallen, die durch die höchste Biomasse der mindestens einen Lemnoideae-Art und der höchsten Bakterienzahl der mindestens einen Bakterienart gekennzeichnet sind, umfasst.

7. Zusammensetzung, umfassend eine mit Vitamin B12 angereicherte Wasserlinsen-Bakterienkultur (Duckweed Bacterial Culture - DBC), wobei die Zusammensetzung mindestens eine Lemnoideae-Art und mindestens eine B12 produzierende Bakterienart umfasst, wobei ferner der Vitamin B12-Gehalt in der Zusammensetzung im Bereich von zwischen 0,01 und 100 µg pro 100 g der DBC liegt.

8. Zusammensetzung nach Anspruch 7, wobei mindestens eins aus Folgendem gilt:
a. der Vitamin B12-Gehalt in der Zusammensetzung ist ein vorbestimmter Prozentsatz von mindestens 20 % des empfohlenen Vitamin B12-Tageswerts (Daily Value - DV);
b. der Vitamin B12-Gehalt entspricht in der Zusammensetzung der empfohlenen Standard-Einnahmemenge von Vitamin B12 (recommended daily intake - RDI) für einen Erwachsenen im Bereich von 0,4 µg bis 3 g am Tag;
c. die mindestens eine B12 produzierende Bakterienart befindet sich in Verbindung mit der mindestens einen Lemnoideae-Art;
d. die Lemnoideae-Art und die B12-produzierende Bakterienart befinden sich in einer Verbindung, wobei die Verbindung ausgewählt ist aus der Gruppe bestehend aus: symbiotischer Wechselbeziehung, persistentem Mutualismus, persistenter biologischer Wechselbeziehung, Mutualismus, gegenseitigem Altruismus zwischen den Arten, kommensalistischer Wechselbeziehung, parasitischer Symbiose, obligater Wechselbeziehung, fakultativer Wechselbeziehung, obligatorisch für beide Arten, obligatorisch für eine, aber fakultativ für die andere, fakultativ für beide Arten, Ektosymbiose, Endosymbiose, kommensalistischer Ektosymbiose, mutualistischer Ektosymbiose, Ektoparasitismus, verbundener Symbiose, nicht verbundener Symbiose, antagonistischer oder antipathischer Symbiose oder Beziehung, nekrotrophischer Wechselbeziehung, biotrophischer Wechselbeziehung, Amensalismus, Konkurrenzbeziehung, antibiotischer Beziehung, Synnekrose und jeder Kombination davon;
e. die B12 produzierende Bakterienart ist ausgewählt aus der Gruppe bestehend aus:
Pseudomonas, *Bacillus, Methanobacterium, Propionibacterium, Acetobacterium, Aerobacter, Agrobacterium, Alcaligenes, Azotobacter, Clostridium, Flavobacterium, Lactobacillus, Micromonospora, Mycobacterium, Nocardia, Protaminobacter, Proteus, Rhizobium, Salmonella, Serratia, Streptomyces, Streptococcus, Xanthomonas* und jeder Kombination davon;
f. die Lemnoideae-Arten enthaltende Biomasse ist ausgewählt aus der Gruppe bestehend aus: Biomasse aus ganzen Lemnoideae, Biomasse aus frischen Lemnoideae, Biomasse aus trockenen Lemnoideae und jeder Kombination davon;
g. die Lemnoideae-Art gehört zu einer Gattung, die ausgewählt ist aus der Gruppe bestehend aus:
*Landoltia, Lemna, Spirodela, Wolffia, Wolffiella und* jeder Kombination davon; und
h. die Lemnoideae-Art ist ausgewählt aus der Gruppe bestehend aus: *Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta* und jeder Kombination davon.

9. Zusammensetzung, umfassend eine mit Vitamin B12 *angereicherte* Wasserlinsen-Bakterienkultur (Duckweed Bacterial Culture - DBC), hergestellt durch das Verfahren nach Anspruch 1.

10. Zusammensetzung nach Anspruch 9, wobei mindestens eins aus Folgendem gilt:
a. die vorbestimmten Bedingungen sind ausgewählt aus der Gruppe bestehend aus Bedingungen für das Wachstum der Biomasse der Lemnoideae-Art, Bedingungen für die Fermentierung und die Vitamin B12-Synthese der Bakterienart und einer Kombination davon;
b. das Volumen von Nährmedien ist eine Batch- oder eine kontinuierliche Kultur;
c. das Volumen von Nährmedien ist ausgewählt aus der Gruppe bestehend aus: einer Wasserpflanzen-Zuchtanlage und einer Mikroben-Zuchtanlage; und
d. das Volumen von Nährmedien ist ausgewählt aus der Gruppe bestehend aus: einem Teich, einer Rinne, einem Aquarium, einem Fermenter, einem Bioreaktor, Kieseln und jedem anderen Substrat.

11. Zusammensetzung nach Anspruch 10, wobei mindestens eins aus Folgendem gilt:
a. die vorbestimmten Bedingungen für das Wachstum der Biomasse der Lemnoideae-Art sind ausgewählt aus der Gruppe bestehend aus: mineralischer Zusammensetzung der Nährmedien, mineralischer Konzentration der Nährmedien, Harnstoffkonzentration, Nitrit- und Nitratkonzentration, Gesamtammoniakkonzentration, Temperaturbereich der Züchtungsmedien, Temperaturbereich der Atmosphäre, Wasserbehandlungsverfahren, Beleuchtungsstärke, Belüftung, Sauerstoffkonzentration, pH-Wert und jeder Kombination davon; und
b. die vorbestimmten Bedingungen für die Fermentation und Vitamin B12-Synthese der Bakterienart sind ausgewählt aus der Gruppe bestehend aus: Zuckerkonzentration im Bereich von 0,01-3,0 % w/v, Aminosäuren und/oder Peptide und/oder Vitaminquellen in einem Konzentrationsbereich von 0,01-3,0 % w/v davon, Aminosäuren oder Mischungen davon in einem Konzentrationsbereich von 0,0001-0,3 g/l, Spurenelemente in einem Konzentrationsbereich von 0,0001-0,3 g/l, Vitaminen und jeder Kombination davon.

12. Zusammensetzung nach Anspruch 11, wobei mindestens eins aus Folgendem gilt:
a. das Mineral ist ausgewählt aus der Gruppe bestehend aus: Mg²⁺, Mn, Zn, Fe²⁺, Zn²⁺, Mn²⁺, CaCl₂ und jeder Kombination davon;
b. das Wasserbehandlungsverfahren ist ausgewählt aus der Gruppe bestehend aus: Denitrifikation, mechanisch, Filtration und jeder Kombination davon;
c. die Zucker sind ausgewählt aus der Gruppe bestehend aus Dextrose, Glucose, Lactose und jeder Kombination davon;
d. die Aminosäuren und/oder Peptide und/oder Vitaminquellen sind ausgewählt aus der Gruppe bestehend aus Hefeextrakt, enzymatischem Abbau von Casein, enzymatischem Abbau von Gelatine und jeder Kombination davon;
e. die Aminosäuren oder deren Mischungen sind ausgewählt aus der Gruppe bestehend aus L-Arginin:HCl, L-Cystein, L-Glutamin, Glycin, L-Histidin:HCl:H₂O, L-Isoleucin, L-Leucin, L-Lysin:HCl, L-Methionin, L-Phenylalanin, L-Serin, L-Threonin, L-Tryptophan, L-Tyrosin:2Na:2H₂O, L-Valin und jeder Kombination davon; und
f. die Spurenelemente sind ausgewählt aus der Gruppe bestehend aus: Cholinchlorid, Folsäure, Myo-Inositol, Niacinamid, D-Calcium-Pantothenat, Calciumchlorid, Eisen(III)Nitrat, Magnesiumsulfat, Kaliumchlorid, Natriumchlorid, Natriumphosphat und jeder Kombination davon.

13. System zum Herstellen einer mit Vitamin B12 angereicherten Wasserlinsen-Bakterienkultur(Duckweed Bacterial Culture - DBC)-Zusammensetzung, wobei das System Folgendes umfasst:
a. mindestens ein Inokulum einer Lemnoideae-Art und mindestens ein Inokulum einer Vitamin B12 produzierenden Bakterienart zur Kultivierung in einem Volumen von Nährmedien;
b. ein Inkubationsmittel zum Inkubieren des Inokulums der mindestens einen Lemnoideae-Art und des Inokulums der mindestens einen Vitamin B12 produzierenden Bakterienart unter vorbestimmten Bedingungen, um eine Wasserlinsen-Bakterienkultur (Duckweed-Bacterial Culture - DBC) bereitzustellen;
c. Mittel zum Bestimmen von Zeitintervallen in den graphischen Darstellungen, die durch DBC mit dem höchsten Vitamin B12-Gehalt gekennzeichnet sind; und
d. Mittel zum Ernten der DBC in den vorbestimmten Zeitintervallen, wodurch eine mit Vitamin B12 angereicherte DBC-Zusammensetzung bereitgestellt wird.

14. System nach Anspruch 13, wobei das System außerdem mindestens ein Mittel zur graphischen Darstellung umfasst, das ausgewählt ist aus der Gruppe bestehend aus:
a. graphischen Darstellungsmitteln zur graphischen Darstellung von Lemnoideae-Pflanzenbiomasse unter den vorbestimmten Bedingungen über die Zeit;
b. graphischen Darstellungsmitteln zur graphischen Darstellung der Bakterienzahl der mindestens einen B12 produzierenden Bakterienart unter den vorbestimmten Bedingungen über die Zeit; und
c. graphischen Darstellungsmitteln zur graphischen Darstellung des Vitamin B12-Gehalts in der DBC unter den vorbestimmten Bedingungen über die Zeit.

15. Zusammensetzung, umfassend einen mit Vitamin B12 angereicherten Extrakt aus mindestens einer Lemnoideae-Art und mindestens einer B12 produzierenden Bakterienart, wobei ferner der Vitamin B12-Gehalt in der Zusammensetzung im Bereich von zwischen 0,01 und 100 µg pro 100 g der Wasserlinsen-Bakterienkultur (Duckweed Bacterial Culture - DBC) liegt.

## Revendications

1. Procédé de production d'une composition de culture bactérienne de lentilles d'eau (DBC) enrichie en vitamine B12, où ledit procédé comprend les étapes de:
a. inoculer au moins une espèce de Lemnoideae et au moins une espèce de bactéries produisant de la vitamine B12 dans un volume de milieu de croissance;
b. Incuber ladite au moins une espèce de Lemnoideae et ladite au moins une espèce de bactéries produisant de la vitamine B12 dans des conditions prédéterminées pour fournir une culture bactérienne de lentilles d'eau (DBC);
c. représenter graphiquement la biomasse végétale de Lemnoideae dans lesdites conditions prédéterminées en fonction du temps;
d. représenter graphiquement le nombre bactérien de ladite au moins une espèce de bactéries produisant B12 dans lesdites conditions prédéterminées en fonction du temps;
e. représenter graphiquement la teneur en vitamine B12 dans ladite DBC dans lesdites conditions prédéterminées en fonction du temps;
f. déterminer des intervalles de temps dans lesdites représentations graphiques **caractérisés par** une DBC avec la plus haute teneur en vitamine B12; et
g. récolter ladite DBC auxdits intervalles de temps prédéterminés, fournissant ainsi une composition de DBC enrichie en vitamine B 12.

2. Procédé selon la revendication 1, dans lequel ladite étape d'incubation comprend en outre au moins une étape de:
a. sélectionner lesdites conditions prédéterminées conçues pour (i) une croissance optimale de ladite au moins une plante de l'espèce de Lemnoideae, et (ii) une fermentation optimale desdites bactéries synthétisant de la vitamine B12 qui lui sont associées;
b. faire croître ladite au moins une espèce de Lemnoideae et ladite au moins une espèce de bactéries produisant de la vitamine B12 dans des conditions prédéterminées pour fournir une association entre ladite au moins une espèce bactérienne et ladite au moins une espèce de Lemnoideae;
c. sélectionner lesdites conditions prédéterminées pour la croissance de biomasse de ladite espèce de Lemnoideae dans le groupe consistant en: la composition minérale du milieu de croissance, la concentration minérale du milieu de croissance, la concentration d'urée, la concentration de nitrites et nitrates, la concentration totale d'ammoniac, la plage de température du milieu de croissance, la plage de température de l'atmosphère, la procédure de traitement de l'eau, l'intensité d'éclairage, l'aération, la concentration d'oxygène, le pH et toute combinaison de celles-ci ; et
d. sélectionner lesdites conditions prédéterminées pour la fermentation symbiotique et la synthèse de vitamine B12 de ladite espèce bactérienne dans le groupe consistant en: la concentration en sucre dans la plage de 0,01-3,0% p/v, les sources d'acides aminés et/ou de peptides et/ou de vitamines dans une plage de concentration de 0,01-3,0% p/v de celles-ci, les acides aminés ou les mélanges de ceux-ci dans une plage de concentration de 0,0001-0,3 g/l, les microéléments dans une plage de concentration de 0,0001-0,3 g/l, les vitamines et toute combinaison de ceux-ci.

3. Procédé selon la revendication 2, comprenant en outre au moins une étape de:
a. sélectionner ladite association dans le groupe consistant en : l'interaction symbiotique, le mutualisme persistant, l'interaction biologique persistante, le mutualisme, l'altruisme réciproque interspécifique, l'interaction commensaliste, la symbiose parasitaire, l'interaction obligatoire, l'interaction facultative, obligatoire pour les deux espèces, obligatoire pour l'une mais facultative pour l'autre, facultative pour les deux espèces, l'ectosymbiose, l'endosymbiose, l'ectosymbiose commensale, ectosymbiose mutualiste, l'ectoparasitisme, la symbiose conjonctive, la symbiose disjonctive, la symbiose ou relation antagoniste ou antipathique, l'interaction nécrotrophique, l'interaction biotrophique, l'amensalisme, la relation de compétition, la relation d'antibiose, la synnécrose et toute combinaison de celles-ci;
b. sélectionner ledit minéral dans le groupe consistant en: Mg²⁺, Mn, Zn, Fe²⁺, Zn²⁺, Mn²⁺, CaCl₂ et toute combinaison de ceux-ci;
c. sélectionner ladite procédure de traitement de l'eau dans le groupe consistant en: dénitrification, filtration mécanique et toute combinaison de celles-ci;
d. sélectionner ledit sucre dans le groupe consistant en dextrose, glucose, lactose et toute combinaison de ceux-ci;
e. sélectionner lesdites sources d'acides aminés et/ou de peptides et/ou de vitamines dans le groupe consistant en extrait de levure, produit de digestion enzymatique de la caséine, produit de digestion enzymatique de la gélatine et toute combinaison de ceux-ci;
f. sélectionner lesdits acides aminés ou leurs mélanges dans le groupe consistant en L-arginineoHCl, L-cystéine, L-glutamine, glycine, L-histidineoHCloH₂O, L-isoleucine, L-leucine, L-lysineoHCl, L-méthionine, L-phénylalanine, L-sérine, L-thréonine, L-tryptophane, L-tyrosine 2Nao2H2O, L-valine et toute combinaison de ceux-ci; et
g. sélectionner lesdits microéléments dans le groupe consistant en : chlorure de choline, acide folique, myo-inositol, niacinamide, acide D-pantothénique hémicalcique, chlorure de calcium, nitrate ferrique, sulfate de magnésium, chlorure de potassium, chlorure de sodium, phosphate de sodium et toute combinaison de ceux-ci.

4. Procédé selon la revendication 1, dans lequel ladite étape d'inoculation comprend au moins une étape de:
a. faire croître ladite au moins une espèce de Lemnoideae et ladite au moins une espèce de bactéries produisant B12 sous forme de lot ou sous forme de culture continue;
b. sélectionner ledit volume de milieu de croissance dans le groupe consistant en : une installation de croissance de plantes aqueuses et une installation de croissance microbienne; et
c. sélectionner ledit volume de milieu de croissance dans le groupe consistant en: un bassin, un canal, un aquarium, un fermenteur, un bioréacteur, des galets et tout autre substrat.

5. Procédé selon la revendication 1, comprenant en outre au moins une étape de:
a. sélectionner lesdites bactéries dans le groupe consistant en : les espèces de *Pseudomonas,* les espèces de *Bacillus,* les espèces de *Methanobacterium,* les espèces de *Propionibacterium, Acetobacterium, Aerobacter, Agrobacterium, Alcaligenes, Azotobacter, Clostridium, Flavobacterium, Lactobacillus, Micromonospora, Mycobacterium, Nocardia, Protaminobacter, Proteus, Rhizobium, Salmonella, Serratia, Streptomyces, Streptococcus, Xanthomonas* et toute combinaison de celles-ci;
b. sélectionner ladite biomasse dans le groupe consistant en: la biomasse de Lemnoideae entière, la biomasse de Lemnoideae fraîche, la biomasse de Lemnoideae sèche et toute combinaison de celles-ci;
c. sélectionner ladite au moins une espèce de Lemnoideae parmi une espèce appartenant au groupe de genres consistant en: *Landoltia, Lemna, Spirodela, Wolffia, Wolffiella* et toute combinaison de celles-ci;
d. sélectionner ladite au moins une espèce de Lemnoideae dans le groupe consistant en : *Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta* et toute combinaison de celles-ci, et
e. fournir une composition de DBC enrichie en vitamine B12 comprenant entre 0,01 et 100 µg de vitamine B12 pour 100 g de ladite DBC.

6. Procédé selon la revendication 1, dans lequel ladite étape de détermination comprend en outre des étapes de détermination des courbes de croissance de ladite au moins une espèce de Lemnoideae et de ladite au moins une espèce de bactéries produisant B12 en fonction du temps et d'identification d'intervalles de temps **caractérisé par** la biomasse la plus élevée de ladite au moins une espèce de Lemnoideae et le plus grand nombre de cellules bactériennes de ladite au moins une espèce de bactéries.

7. Composition comprenant une culture bactérienne de lentilles d'eau (DBC) enrichie en vitamine B12, dans laquelle ladite composition comprend au moins une espèce de Lemnoideae et au moins une espèce de bactéries produisant B12, dans laquelle en outre ladite teneur en vitamine B12 dans ladite composition est dans la plage entre 0,01 et 100 µg pour 100g de ladite DBC.

8. Composition selon la revendication 7, dans laquelle au moins l'une des suivantes est vraie:
a. ladite teneur en vitamine B12 dans ladite composition est un pourcentage prédéterminé d'au moins 20% de la valeur quotidienne recommandée (DV) de vitamine B12;
b. ladite teneur en vitamine B12 dans ladite composition est conforme à la norme de l'apport quotidien recommandé (RDI) en vitamine B12 pour un adulte allant de 0,4 µq à 3 µg par jour;
c. ladite au moins une espèce de bactéries produisant B12 est en association avec ladite au moins une espèce de Lemnoideae;
d. ladite espèce de Lemnoideae et ladite espèce de bactéries produisant B12 sont en association, ladite association sélectionnée dans le groupe consistant en: l'interaction symbiotique, le mutualisme persistant, l'interaction biologique persistante, le mutualisme, l'altruisme réciproque interspécifique, l'interaction commensaliste, la symbiose parasitaire, l'interaction obligatoire, l'interaction facultative, obligatoire pour les deux espèces, obligatoire pour l'une mais facultative pour l'autre , facultative pour les deux espèces, l'ectosymbiose, l'endosymbiose, l'ectosymbiose commensale, ectosymbiose mutualiste, l'ectoparasitisme, la symbiose conjonctive, la symbiose disjonctive, la symbiose ou relation antagoniste ou antipathique, l'interaction nécrotrophique, l'interaction biotrophique, l'amensalisme, la relation de compétition, la relation d'antibiose, la synnécrose et toute combinaison de celles-ci;
e. ladite espèce de bactérie produisant B12 est sélectionnée dans le groupe consistant en : les espèces de *Pseudomonas,* les espèces de *Bacillus,* les espèces de *Methanobacterium,* les espèces de *Propionibacterium, Acetobacterium, Aerobacter, Agrobacterium, Alcaligenes, Azotobacter, Clostridium, Flavobacterium, Lactobacillus, Micromonospora, Mycobacterium, Nocardia, Protaminobacter, Proteus, Rhizobium, Salmonella, Serratia, Streptomyces, Streptococcus, Xanthomonas* et toute combinaison de celles-ci;
f. ladite biomasse contenant une espèce de Lemnoideae est choisie dans le groupe consistant en: la biomasse de Lemnoideae entière, la biomasse de Lemnoideae fraîche, la biomasse Lemnoideae sèche et toute combinaison de celles-ci;
g. ladite espèce de Lemnoideae appartient à un genre choisi dans le groupe consistant en: *Landoltia, Lemna, Spirodela, Wolffia, Wolffiella* et toute combinaison de celles-ci; et
h. ladite espèce de Lemnoideae est choisie dans le groupe consistant en : *Wolffia angusta, Wolffia arrhiza, Wolffia australiana, Wolffia borealis, Wolffia brasiliensis, Wolffia Columbiana, Wolffia cylindracea, Wolffia elongate, Wolffia globose, Wolffia microscopica, Wolffia neglecta* et toute combinaison de celles-ci.

9. Composition comprenant une culture bactérienne de lentilles d'eau (DBC) enrichie en vitamine B12 produite par le procédé selon la revendication 1.

10. Composition selon la revendication 9, dans laquelle au moins l'une des suivantes est vraie:
a. lesdites conditions prédéterminées sont choisies dans le groupe consistant en les conditions pour la croissance de la biomasse de ladite espèce de Lemnoideae, les conditions pour la fermentation et la synthèse de vitamine B12 de ladite espèce bactérienne et une combinaison de celles-ci;
b. ledit volume de milieu de croissance est un lot ou une culture continue;
c. ledit volume de milieu de croissance est choisi dans le groupe consistant en: une installation de croissance de plantes aqueuses et une installation de croissance microbienne; et
d. ledit volume de milieu de croissance est choisi dans le groupe consistant en: un bassin, un canal, un aquarium, un fermenteur, un bioréacteur, des galets et tout autre substrat.

11. Composition selon la revendication 10, dans laquelle au moins l'une des suivantes est vraie:
a. lesdites conditions prédéterminées pour la croissance de biomasse de ladite espèce de Lemnoideae sont choisies dans le groupe consistant en : la composition minérale du milieu de croissance, la concentration minérale du milieu de croissance, la concentration d'urée, la concentration de nitrites et nitrates, la concentration totale d'ammoniac, la plage de température du milieu de croissance, la plage de température de l'atmosphère, la procédure de traitement de l'eau, l'intensité d'éclairage, l'aération, la concentration d'oxygène, le pH et toute combinaison de celles-ci; et
b. lesdites conditions prédéterminées pour la fermentation symbiotique et la synthèse de vitamine B12 de ladite espèce bactérienne sont choisies dans le groupe consistant en: la concentration en sucre dans la plage de 0,01-3,0% p/v, les sources d'acides aminés et/ou de peptides et/ou de vitamines dans une plage de concentration de 0,01-3,0% p/v de celles-ci, les acides aminés ou les mélanges de ceux-ci dans une plage de concentration de 0,0001-0,3 g/l, les microéléments dans une plage de concentration de 0,0001-0,3 g/l, les vitamines et toute combinaison de ceux-ci.

12. Composition selon la revendication 11, dans laquelle au moins l'une des suivantes est vraie:
a. ledit minéral est sélectionné dans le groupe consistant en: Mg²⁺, Mn, Zn, Fe²⁺, Zn²⁺, Mn²⁺, CaCl₂ et toute combinaison de ceux-ci ;
b. ladite procédure de traitement de l'eau est sélectionnée dans le groupe consistant en: dénitrification, filtration mécanique et toute combinaison de celles-ci;
c. lesdits sucres sont sélectionnés dans le groupe consistant en dextrose, glucose, lactose et toute combinaison de ceux-ci;
d. lesdites sources d'acides aminés et/ou de peptides et/ou de vitamines sont sélectionnées dans le groupe consistant en extrait de levure, produit de digestion enzymatique de la caséine, produit de digestion enzymatique de la gélatine et toute combinaison de ceux-ci;
e. lesdits acides aminés ou leurs mélanges sont sélectionnés dans le groupe consistant en L-arginineoHCl, L-cystéine, L-glutamine, glycine, L-histidineoHCloH₂O, L-isoleucine, L-leucine, L-lysineoHCl, L-méthionine, L-phénylalanine, L-sérine, L-thréonine, L-tryptophane, L-tyrosine 2Nao2H2O, L-valine et toute combinaison de ceux-ci; et
f. lesdits microéléments sont sélectionnés dans le groupe consistant en : chlorure de choline, acide folique, myo-inositol, niacinamide, acide D-pantothénique hémicalcique, chlorure de calcium, nitrate ferrique, sulfate de magnésium, chlorure de potassium, chlorure de sodium, phosphate de sodium et toute combinaison de ceux-ci.

13. Système pour produire une composition de culture bactérienne de lentilles d'eau (DBC) enrichie en vitamine B12 dans lequel ledit système comprend:
a. au moins un inoculum d'espèce de Lemnoideae et au moins un inoculum d'espèce de bactéries produisant de la vitamine B12 pour la culture dans un volume de milieu de croissance;
b. un moyen d'incubation pour incuber ledit au moins un inoculum d'espèce de Lemnoideae et ledit au moins un inoculum d'espèce de bactéries produisant de la vitamine B12 dans des conditions prédéterminées pour fournir une culture bactérienne de lentilles d'eau (DBC);
c. des moyens pour déterminer des intervalles de temps dans lesdites représentations graphiques **caractérisés par** une DBC avec la plus haute teneur en vitamine B12; et
d. des moyens pour récolter ladite DBC auxdits intervalles de temps prédéterminés, fournissant ainsi une composition de DBC enrichie en vitamine B12.

14. Système selon la revendication 13, dans lequel ledit système comprend en outre au moins un moyen de représentation graphique choisi dans le groupe consistant en:
a. les moyens de représentation graphique pour représenter graphiquement la biomasse végétale de Lemnoideae dans lesdites conditions prédéterminées en fonction du temps;
b. les moyens de représentation graphique pour représenter graphiquement le nombre bactérien de ladite au moins une espèce de bactéries produisant B12 dans lesdites conditions prédéterminées en fonction du temps; et
c. les moyens de représentation graphique pour représenter graphiquement la teneur en vitamine B12 dans ladite DBC dans lesdites conditions prédéterminées en fonction du temps.

15. Composition comprenant un extrait enrichi en vitamine B12 d'au moins une espèce de Lemnoideae et d'au moins une espèce de bactéries produisant B12, en outre dans lequel ladite teneur en vitamine B12 dans ladite composition est dans la plage entre 0,01 et 100 µg pour 100 g de culture bactérienne de lentilles d'eau (DBC).
